# FASCICULE DE BREVET EUROPEEN

(11) **EP 3 384 047 B1**
(45) Date de publication et mention de la délivrance du brevet: **27.01.2021**
(21) Numéro de dépôt: 16819968.5
(22) Date de dépôt: 01.12.2016
(51) Int. Cl.: C12Q 1/6883

(54) **PROCEDE D'EVALUATION DU RISQUE DE COMPLICATIONS CHEZ LES PATIENTS QUI PRESENTENT UN SYNDROME DE REPONSE INFLAMMATOIRE SYSTEMIQUE (SIRS)**
VERFAHREN ZUR BEWERTUNG DES KOMPLIKATIONSRISIKOS BEI PATIENTEN MIT SYSTEMISCHEM INFLAMMATORISCHEM RESPONSE-SYNDROM (SIRS)
METHOD FOR ASSESSING THE RISK OF COMPLICATIONS IN PATIENTS WITH SYSTEMIC INFLAMMATORY RESPONSE SYNDROME (SIRS)

(30) Priorité: 01.12.2015 FR 1561671
(43) Date de publication de la demande: 10.10.2018
(73) Titulaire: bioMérieux, 69280 Marcy-l'Étoile (FR); Hospices Civils De Lyon, 69229 Lyon Cedex 02 (FR); Université Claude Bernard Lyon I, 69100 Villeurbanne (FR)
(72) Inventeur: CERRATO, Elisabeth, 69007 Lyon (FR); DELWARDE, Benjamin, 69007 Lyon (FR); MONNERET, Guillaume, 69008 Lyon (FR); PERONNET, Estelle, 69009 Lyon (FR); TEXTORIS, Julien, 69100 Villeurbanne (FR); VENET, Fabienne, 69008 Lyon (FR)
(74) Mandataire: Cabinet Beau de Loménie
(86) Numéro de dépôt international: PCT/FR2016/053164
(87) Numéro de publication internationale: WO 2017/093672

(56) Documents cités:
- WO-A1-2015/040328
- US-A1- 2011 098 195
- MICHAEL BAUER ET AL: "A Transcriptomic Biomarker to Quantify Systemic Inflammation in Sepsis - A Prospective Multicenter Phase II Diagnostic Study", EBIOMEDICINE, vol. 6, 8 mars 2016 (2016-03-08), pages 114-125, XP055290123, ISSN: 2352-3964, DOI: 10.1016/j.ebiom.2016.03.006
- WILLEMIJN M. PASSTOORS ET AL: "IL7R gene expression network associates with human healthy ageing", IMMUNITY & AGEING, vol. 25, no. 1, 11 novembre 2015 (2015-11-11), page 402, XP055290637, DOI: 10.1186/s12979-015-0048-6

## Description

La présente invention concerne le domaine médical en général, et en particulier le domaine de la prédiction de risques. Plus précisément, l'invention concerne un procédé d'évaluation du risque de complication chez un patient ayant subi une agression, telle que chirurgie, brûlure, traumatisme..., générant un syndrome de réponse inflammatoire systémique ou SIRS ou chez un patient connaissant une infection provoquant un SIRS, et en particulier chez un patient en état septique, c'est-à-dire un patient présentant un sepsis, notamment un sepsis sévère, également nommé sepsis grave, et de préférence chez un patient en choc septique ou ayant subi un choc septique.

Dans le cas d'un syndrome de réponse inflammatoire systémique ou SIRS, les risques de complications, du type infection secondaire ou infection nosocomiale dans le cas d'une hospitalisation, et le risque de décès sont importants.

Les infections nosocomiales, notamment, sont un problème de santé publique important. Les patients hospitalisés ont souvent, par nature, des défenses immunitaires diminuées ou altérées, du fait de pathologies portant directement atteinte à leurs compétences immunitaires, ou en raison de leur état général. Ces patients, et en particulier les personnes dénutries ou aux âges extrêmes de la vie (personnes âgées, nourrissons) sont spécialement sensibles aux infections en général, et en particulier à la survenue d'infections nosocomiales. La prévalence des infections nosocomiales est nettement plus élevée dans les unités de réanimation que dans d'autres secteurs hospitaliers.

Par ailleurs, parmi les syndromes de réponses inflammatoires systémiques ou SIRS, le sepsis est un syndrome de réponse systémique inflammatoire en relation avec une infection. Un sepsis sévère est un sepsis associé à une hypotension artérielle et/ou hypoperfusion et/ou au dysfonctionnement d'au moins un organe. Le choc septique est un sepsis sévère associé à une hypotension persistante malgré des remplissages adéquats et des traitements vasopresseurs. La différence entre sepsis, sepsis sévère et choc septique, réside principalement dans l'importance de la perturbation de toutes les fonctions vitales.

Les patients présentant un SIRS, et en particulier ceux présentant des syndromes septiques, c'est-à-dire les patients en état septique allant de sepsis, sepsis sévère, à choc septique, présentent un risque important de complications, notamment d'infections nosocomiales. De plus, les états septiques correspondent à l'une des premières causes de mortalité dans les services de réanimation.

Estimer le risque de complication d'un patient admis en service de réanimation ou dans d'autres services, par exemple en cas de chirurgie, notamment une chirurgie lourde ou transplantation, qui présente un SIRS, et en particulier, un sepsis, un sepsis sévère ou en état de choc septique, est donc essentiel pour pouvoir proposer une prise en charge personnalisée, et ainsi tenter de réduire les risques de complication, et en particulier de décès.

La sévérité de l'état d'un patient admis en service de réanimation est généralement estimée à l'aide de différents paramètres cliniques et physiologiques. Ceux-ci permettent notamment de définir des scores prédictifs en termes de survie/mortalité, parmi lesquels on peut notamment citer les scores de sévérité SOFA pour *Sequential Organ Failure Assessment* ou *Sepsis-related Organ Failure Assessment* (Vincent *et al.,* 1996) et SAPSII pour *Simplified Acute Physiology Score II,* aussi nommé en français IGS II pour Indice de Gravité Simplifié II (Le Gall *et al.,* 1993). Ces scores composites, définis sur des cohortes conséquentes de patients de réanimation, incluent plusieurs paramètres clinico-biologiques tels que le nombre de plaquettes circulantes, la bilirubinémie, la diurèse, l'âge ou la température corporelle. Ces scores permettent d'évaluer par le calcul d'une valeur numérique le degré d'atteinte de la fonction d'un ou de plusieurs systèmes physiologiques (par exemple : cardiovasculaire, rénal, cérébral). Ils sont calculés durant les premiers jours d'admission en réanimation. Dans le cas du score SAPSII, seule la valeur la plus délétère des paramètres inclus dans le score, mesurée au cours des 24 premières heures de séjour en réanimation, est prise en compte.

Ces scores sont néanmoins peu pratiques en routine clinique car ils nécessitent, de la part du médecin, une démarche active de recherche des paramètres cliniques dans le dossier du patient.

Ainsi, il existe un réel besoin de disposer d'autres outils, et notamment de marqueurs mesurables, permettant d'évaluer facilement et rapidement le risque de complication, et en particulier de mortalité d'un patient admis en service de réanimation qui est par définition dans un état grave, et chez lequel le pronostic vital peut éventuellement être rapidement engagé. En effet, pouvoir identifier les sujets dont le risque de complication, voire de mortalité est accru permettrait de leur réserver une prise en charge, un suivi, ainsi que des thérapeutiques plus adaptées.

La demande WO 2013/140103 propose un procédé de détermination de la susceptibilité aux infections nosocomiales chez un patient, comprenant les étapes suivantes :
- mesurer l'expression de sCD127 au niveau protéique dans un échantillon biologique issu dudit patient ou échantillon à tester,
- conclure à une susceptibilité accrue aux infections nosocomiales après comparaison de l'expression de sCD127 par rapport à une valeur de référence.

La demande WO 2015/040328 décrit, quant à elle, un procédé d'évaluation du risque de mortalité chez un patient ayant subi une agression ou une infection générant une réponse inflammatoire systémique, comprenant les étapes suivantes :
- mesurer l'expression de sCD127 au niveau protéique dans un échantillon biologique issu dudit patient, également nommé échantillon à tester,
- conclure à un risque accru de mortalité après comparaison de l'expression de sCD127 par rapport à une valeur de référence.

Le sCD127 est la forme soluble ou plasmatique du CD127, encore nommé chaîne alpha du récepteur de l'IL-7 ou IL-7R-ALPHA ou IL-7RA ou CDw127 (UniProtKB P16871). Le CD127 est une glycoprotéine de 75 kDa membre de la superfamille des récepteurs aux facteurs de croissance hématopoïétiques. Il est exprimé au niveau membranaire et s'associe avec le CD132 (chaîne γ_{c} commune) pour former le récepteur de l'IL-7. Ce récepteur joue un rôle important dans la différenciation, la survie et la prolifération lymphocytaire. Le CD127 est constitué d'une partie extracellulaire de 219 acides aminés (aa), d'une partie transmembranaire de 25 aa et d'une partie intracytoplasmique de 195 aa (Jiang *et al.,* 2005). Comme pour de nombreux récepteurs de cytokines, il a été montré que le CD127 pouvait être présent dans le plasma ou le sérum sous forme soluble, nommée « sCD127 » (Carini *et al.,* 1994; Vranjkovic *et al.,* 2007). Par « sCD127 », on entend la forme soluble ou forme circulante (encore nommée forme plasmatique ou sérique) du récepteur de l'IL-7. Les mécanismes à l'origine de la libération de la forme soluble sont peu décrits et les résultats de la littérature sont contradictoires. Ainsi, les travaux de Vranjkovic *et al.,* 2007 conduisent à la conclusion que la forme soluble proviendrait d'un clivage de la forme membranaire. Au contraire, Goodwin *et al.,* 1990 ainsi que Rose *et al.,* 2009, concluent à une régulation de la transcription, *via* un épissage alternatif de l'ARN du gène *IL7R* codant pour CD127.

La demande WO 2009/115478 décrit, quant à elle, une méthode de détection et de différenciation *in vitro* de différents états physiopathologiques. La méthode de détection décrite dans ce document est notamment définie à la revendication 1 et utilise la détection de plusieurs marqueurs polynucléotidiques. Différents états physiopathologiques dont le SIRS, le sepsis, le choc septique sont cités. Mais il est question de détection *in vitro,* de différenciation ou de l'observation d'une progression et non de l'évaluation d'un risque concernant donc un état futur. Le gène de l'IL7R est cité parmi 669 polynucléotidiques. Dans les exemples (voir tableau 10, 11, 13b, 15, 17, 18a et 18b), les marqueurs sont utilisés en tant que marqueurs d'identification de sujets en état de SIRS ou sepsis, comme cela ressort du titre de l'exemple 3 notamment. Ces exemples ne concernent nullement un procédé d'évaluation *in vitro* ou *ex vivo* du risque de complication chez un patient ayant subi une agression ou infection, mais le diagnostic de SIRS ou sepsis.

Le gène *IL7R* est composé de 8 exons, l'exon 6 codant pour le domaine transmembranaire. En particulier, la séquence nucléique de référence pour le gène *IL7R* est la **SEQ ID N°1** (Ensembl : ENSG00000168685).

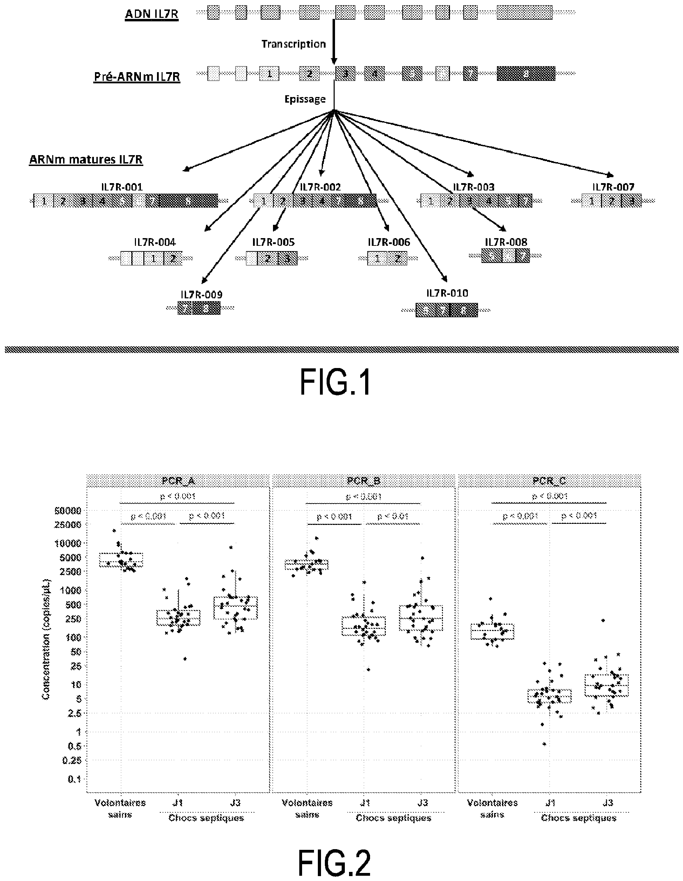

Il existe plusieurs transcrits du gène *IL7R,* recensés dans la base Ensembl (GRCh38.p3), représentés sur la **Figure 1** et listés dans le **Tableau 1.** Le transcrit IL7R-001 comprend l'exon 6 et correspond donc à la forme protéique membranaire de CD127. Tous les autres transcrits pouvant théoriquement aboutir à la traduction d'une protéine ne comprennent pas l'exon 6 et correspondent donc potentiellement à des formes solubles de CD127. **Tableau 1. Les différents transcrits du gène *IL7R* d'après la base Ensembl (GRCh38.p3)**

| **Nom du transcrit** | **Numéro d'identification** | **SEQ ID N°** | **Taille théorique protéine** | **Observation expérimentale de la protéine** |
|---|---|---|---|---|
| **IL7R-001** | ENST00000303115 NM_002185 | **SEQ ID N°2** | 459 aa | Oui (Goodwin *et al.,* 1990; Park *et al.,* 1990) |
| **IL7R-002** | ENST00000514217 | **SEQ ID N°3** | 180 aa | Non (Rose *et al.,* 2009) |
| **IL7R-003** | ENST00000506850 | **SEQ ID N°4** | 261 aa | Oui (Rose *et al.,* 2009) |
| **IL7R-004** | ENST00000508941 | **SEQ ID N°5** | 59 aa | Non |
| **IL7R-005** | ENST00000511031 | **SEQ ID N°6** | Non codant | Non |
| **IL7R-006** | ENST00000515665 | **SEQ ID N°7** | 52 aa | Non |
| **IL7R-007** | ENST00000511982 | **SEQ ID N°8** | 150 aa | Non |
| **IL7R-008** | ENST00000509668 | **SEQ ID N°9** | Non codant | Non |
| **IL7R-009** | ENST00000505875 | **SEQ ID N°10** | Non codant | Non |
| **IL7R-010** | ENST00000505093 | **SEQ ID N°11** | 65 aa | Non |

L'étude de Rose *et al.,* 2009 a notamment permis de montrer que la séquence de la forme protéique soluble sCD127 purifiée à partir de plasma correspondait à la forme obtenue suite à la traduction du transcrit IL7R-003. Selon cette étude, la protéine soluble ne provenait pas de la traduction du transcrit IL7R-002, ni d'un clivage de la forme protéique membranaire.

Jusqu'à ce jour, des formes protéiques solubles correspondant aux transcrits pouvant potentiellement être traduits, IL7R-002, IL7R-004, IL7R-006, IL7R-007 et IL7R-010, n'ont pas été observées expérimentalement.

Dans le cadre de l'invention, les inventeurs de la présente demande de brevet proposent un procédé pour l'évaluation *in vitro* ou *ex vivo* du risque de complication chez un patient ayant subi une agression ou une infection qui adopte une autre solution que la détection de protéines solubles, qui est basée sur l'évaluation d'un ou plusieurs transcrits du gène *IL7R.* Les inventeurs ont démontré que dans le cas des transcrits, il n'était pas utile, ni préféré de se limiter aux transcrits codant pour la partie soluble sCD127 du CD127.

C'est dans ce contexte que la présente invention se propose de fournir un nouveau biomarqueur prédictif d'un risque de complication, et notamment de mortalité, chez un patient ayant subi une agression sévère (chirurgie, brûlure, traumatisme, ...) ou infection, ladite agression ou infection générant un syndrome de réponse inflammatoire systémique (SIRS). L'étude de la quantité d'un ou plusieurs transcrits du gène *IL7R* permet ainsi d'évaluer, facilement et rapidement, le risque de complication et, en particulier, de mortalité du patient et de prendre toutes les dispositions préventives possibles. L'invention concerne un procédé d'évaluation *in vitro* ou *ex vivo* du risque de complication chez un patient ayant subi une agression ou une infection, ladite agression ou infection générant un syndrome de réponse inflammatoire systémique, dans lequel la détection, de préférence la quantification, dans un échantillon biologique issu dudit patient, d'au moins un transcrit du gène *IL7R* est réalisée.

Au sens de l'invention, on entend par « syndrome de réponse inflammatoire systémique » ou « SIRS », une réponse associant au moins deux des critères suivants : Température > 38 °C ou < 36 °C, Fréquence cardiaque > 90/minute, Fréquence respiratoire > 20/minute ou paCO₂ <32 mmHg, Leucocytes > 12000/mm³ ou < 4000/mm³ (Bone *et al.,* 1992). Un SIRS peut être dû à une infection ou à un autre type d'agression du type brûlure, chirurgie ou traumatisme, notamment. Les sepsis, sepsis sévères et chocs septiques correspondent tous à un SIRS dû à une infection. Chez ces patients en état septique (sepsis, sepsis sévère et choc septique), qui présentent donc un SIRS suite à une infection, ladite infection ayant provoqué le SIRS peut être de diverses origines, et en particulier d'origine bactérienne, virale ou fongique. Dans le cas des sepsis sévères et chocs septiques, le SIRS s'accompagne d'au moins une autre manifestation, qui est dans le cas des sepsis sévères, une hypotension artérielle et/ou une hypoperfusion et/ou un dysfonctionnement d'au moins un organe, au(x)quel(s) s'additionne, dans le cas d'un choc septique, une hypotension persistante malgré des remplissages adéquats et requérant l'utilisation de traitements vasopresseurs.

Un patient présentant un SIRS est généralement admis en réanimation lorsque son état implique un suivi continu des fonctions vitales et, le cas échéant, le recours à des méthodes de suppléance (transfusion de dérivés sanguins, remplissage vasculaire, ventilation mécanique, catécholamines, hémodialyse, circulation extracorporelle, etc.). L'objectif final de la réanimation est la restauration de l'homéostasie.

Par « complication », on entend une infection, autre que celle ayant pu causer le syndrome de réponse inflammatoire systémique, ladite infection étant alors nommée secondaire, ou bien une infection primaire lorsque le syndrome de réponse inflammatoire systémique est causé par une agression autre qu'une infection, ou encore on entend par « complication », le décès du patient. La dite infection primaire ou secondaire peut être nosocomiale ou non. Les infections nosocomiales sont exclusivement contractées dans le cas d'une hospitalisation et apparaissent au moins 48 heures après ladite hospitalisation.

Par « risque de complication », on entend la susceptibilité d'un patient à générer une infection primaire ou secondaire, ou la susceptibilité que le patient a à décéder. La présence d'un risque de complication correspond au risque que la complication intervienne, par exemple dans les 60 jours, en particulier dans les 40 jours, notamment dans les 30 jours, suivant l'admission en réanimation d'un patient atteint d'un SIRS ou le début du choc septique dans le cas d'un patient en état de choc septique ou ayant été en état de choc septique, et notamment, ou encore correspond au risque que la complication intervienne pendant toute la durée du séjour en réanimation, voire d'hospitalisation.

Le procédé selon l'invention peut donc être un procédé d'évaluation de la susceptibilité du patient à générer une infection (qui sera primaire dans le cas d'un patient ayant subi une agression, et non une infection, ayant généré le syndrome de réponse inflammatoire systémique et qui sera secondaire dans le cas d'un patient ayant subi une première infection à l'origine du syndrome de réponse inflammatoire systémique). Dans ce cas, le procédé selon l'invention permettra de délivrer une conclusion quant à la présence ou non d'un risque pour le patient de générer une telle infection.

Le procédé selon l'invention est un procédé mis en œuvre *in vitro* ou *ex vivo.* Il présente l'avantage de pouvoir évaluer facilement le risque de complication, et en particulier de mortalité, notamment d'un patient qui est admis en service de réanimation, en disposant d'un marqueur directement mesurable, contrairement aux scores de sévérité SOFA et SAPSII, par exemple, et dont la mesure peut être faite dans un laboratoire de proximité ou au lit du patient. La mesure de la quantité d'un ou plusieurs transcrits du gène *IL7R* est tout à fait adaptée pour être réalisée par des automates d'analyse ou par des tests rapides.

De manière préférée, ledit patient est en état septique, notamment en sepsis sévère ou en choc septique. Cet état correspond à l'état du patient au moment du prélèvement de l'échantillon à tester ou à un état dans lequel le patient a été, de manière rapprochée dans le temps, par rapport au prélèvement de l'échantillon à tester, notamment dans les 6 jours précédant le prélèvement de l'échantillon à tester. Dans le cas d'un sepsis, sepsis sévère ou choc septique notamment, il est possible que le patient soit dans cet état, notamment lors de son admission en service de réanimation et que le sepsis, sepsis sévère ou choc septique ait cessé au moment du prélèvement de l'échantillon à tester. Dans la suite de la description, on dira alors que dans ce cas, le patient a subi ou a été en état de choc septique, sepsis ou sepsis sévère selon le cas. En particulier, dans le procédé selon l'invention, l'échantillon biologique à tester est issu d'un patient présentant un sepsis, notamment un sepsis sévère ou ayant présenté un sepsis, notamment un sepsis sévère dans les 6 jours précédant le prélèvement de l'échantillon biologique à tester ou bien l'échantillon biologique à tester est issu d'un patient en état de choc septique ou ayant été en état de choc septique dans les 6 jours précédant le prélèvement de l'échantillon biologique à tester.

Le procédé selon l'invention est également parfaitement adapté à des patients avec un SIRS, dû à une agression autre qu'une infection, notamment une chirurgie, une brûlure ou un traumatisme.

De manière préférée, le risque de complication évalué dans le cadre de l'invention est le risque de décès du patient. Le procédé selon l'invention peut donc être un procédé d'évaluation de la susceptibilité du patient à décéder. Dans ce cas, le procédé selon l'invention permettra de délivrer une conclusion quant à la présence ou non d'un risque pour le patient de décéder. Dans le cas de la prédiction du risque de décès, ce risque sera évalué pour un décès intervenant dans les 60 jours, en particulier dans les 40 jours, notamment dans les 30 jours, en particulier dans les 28 jours, suivant l'admission du patient en service de réanimation et notamment, pour un décès susceptible d'intervenir pendant toute la durée du séjour en réanimation, voire d'hospitalisation. Comme dit précédemment, la présente invention présente une application préférée chez les patients qui présentent un sepsis, notamment un sepsis sévère ou encore chez les patients en choc septique ou ayant subi un choc septique. De manière préférée, le procédé selon la présente invention est utilisé pour évaluer le risque de mortalité chez de tels patients. De manière particulièrement préférée, le procédé selon la présente invention est tout particulièrement avantageux pour évaluer le risque de mortalité chez un patient qui est ou a été en état de choc septique. Dans ce cas, le risque de décès sera évalué pour un décès intervenant dans les 60 jours, en particulier dans les 40 jours, notamment dans les 30 jours, en particulier dans les 28 jours suivant le début du choc septique, et notamment, pour un décès susceptible d'intervenir pendant toute la durée du séjour en réanimation, voire d'hospitalisation.

De manière préférée dans le cadre de l'invention, ledit au moins un transcrit du gène *IL7R* qui est détecté et, de préférence quantifié, est choisi parmi les transcrits IL7R-001 de **SEQ ID N°2,** IL7R-002 de **SEQ ID N°3,** IL7R-003 de **SEQ ID N°4,** IL7R-005 de **SEQ ID N°6** et IL7R-007 de **SEQ ID N°8** et leurs variants, la séquence d'un variant présentant au moins 99% d'identité avec l'une desdites séquences. Le pourcentage d'identité est déterminé grâce à un logiciel d'alignement de séquences, tel que CLUSTALW (Nucleic Acids Res. 1994 Nov 11;22(22):4673-80. CLUSTAL W: improving the sensitivity of progressive multiple sequence alignment through sequence weighting, position-specific gap penalties and weight matrix choice. Un variant correspondra, en particulier, à un polymorphisme de la séquence du gène *IL7R.* En particulier, ledit au moins un transcrit du gène *IL7R* est choisi parmi les transcrits comprenant au moins une partie du domaine transmembranaire, voire tout le domaine transmembranaire, de CD127, et correspond, de préférence au transcrit IL7R-001 de **SEQ ID N°2** ou à un de ses variants présentant au moins 99% d'identité avec ladite séquence. Le transcrit IL7R-001 de **SEQ ID N°2** a l'avantage d'être détecté en quantité importante, par rapport à d'autres transcrits, ce qui facilite la mise en œuvre du procédé selon l'invention, dans le cas de la détection d'au moins ce transcrit.

Par « transcrit », on entend les ARN, et en particulier les ARN messagers issus, de la transcription du gène *IL7R.* Plus précisément, les transcrits sont les ARN produit par l'épissage du gène. Dans le cadre de l'invention le ou les transcrits du gène *IL7R* détecté(s) et/ou quantifié(s) est(sont) donc, de préférence, un ARNm.

En particulier, un procédé selon l'invention met en œuvre les étapes consistant à :
i) déterminer la quantité dudit au moins un transcrit du gène *IL7R* dans ledit échantillon biologique du patient, nommé échantillon à tester,
ii) comparer la quantité dudit au moins un transcrit déterminée pour ledit échantillon biologique ou une valeur dérivée de cette quantité, à une valeur de référence prédéterminée, et
iii) établir une conclusion quant à la présence éventuelle d'un risque de complication, à partir du résultat de la comparaison.

Il est possible, dans le cadre de l'invention, de détecter et quantifier plusieurs transcrits du gène *IL7R,* et en particulier de détecter et quantifier les transcrits IL7R-001 de **SEQ ID N°2,** IL7R-002 de **SEQ ID N°3,** IL7R-003 de **SEQ ID N°4,** IL7R-005 de **SEQ ID N°6** et IL7R-007 de **SEQ ID N°8.**

Si dans le cadre de l'invention, plusieurs transcrits du gène *IL7R* sont détectés, le procédé mettra, notamment, en œuvre les étapes consistant à :
i) déterminer la quantité globale de plusieurs transcrits du gène *IL7R* dans ledit échantillon biologique du patient,
ii) comparer la quantité globale desdits transcrits déterminée pour ledit échantillon biologique ou une valeur dérivée de cette quantité, à une valeur de référence prédéterminée, et
iii) établir une conclusion quant à la présence éventuelle d'un risque de complication, à partir du résultat de la comparaison.

Dans le cadre de l'invention, au moins un transcrit du gène *IL7R,* est détecté dans un échantillon biologique issu d'un patient, pour lequel on souhaite évaluer un risque de complication, nommé échantillon à tester. Le résultat issu de cette détection va être comparée à une valeur de référence, afin d'évaluer les risques de complication.

Ainsi, dans tous les cas, en plus de la mesure de la quantité dudit au moins un transcrit du gène *IL7R,* proprement dite, dans l'échantillon à tester, le procédé de l'invention peut comprendre l'obtention préalable de la valeur de référence, à laquelle la quantité dudit au moins un transcrit déterminée qui sera détectée dans l'échantillon à tester ou une valeur dérivée de cette quantité pourra être comparée, afin de conclure quant au risque de complication, chez le patient duquel est issu l'échantillon à tester. Il est également possible que cette valeur de référence soit disponible, dans le futur, lors de la mise en œuvre du procédé de l'invention.

La valeur de référence sera déterminée, à partir du ou des mêmes transcrits du gène *IL7R,* que ceux ou celui détecté(s) ou quantifié(s) dans l'échantillon biologique à tester. Elle pourra cependant être déterminée sur un échantillon biologique différent mais du même type, issu du même patient ou d'un autre patient ou pool de patients. Lorsqu'un ou des échantillons provenant d'un patient de référence ou pool de patients de référence seront utilisés pour déterminer la valeur de référence, ils auront, de préférence, le même type de SIRS (notamment sepsis, sepsis sévère ou choc septique) que le patient pour lequel on souhaite mettre en oeuvre le procédé.

L'échantillon à tester dans le cadre du procédé de l'invention est un échantillon biologique provenant du patient chez qui on souhaite évaluer le risque de complication. En particulier, un tel échantillon biologique est choisi parmi ceux susceptibles de contenir des transcrits du gène *IL7R.* Par exemple, l'échantillon à tester provient d'un prélèvement obtenu dans les 6 jours ou à 6 jours (J6) après l'admission en service de réanimation d'un patient avec un SIRS, de préférence dans les 5 jours ou à 5 jours (J5) après l'admission en service de réanimation, de manière préférentielle dans les 4 jours ou à 4 jours (J4) après l'admission en service de réanimation, de manière encore préférée dans les 3 jours ou à 3 jours (J3) après l'admission en service de réanimation, ou encore dans les deux jours ou à 2 jours (J2) après l'admission en service de réanimation, ou encore dans les 24h ou à 24h (J1) après l'admission en service de réanimation. Dans le cas où le SIRS correspond à un choc septique, ces délais seront plutôt calculés à compter du début du choc septique, qui peut être défini par le début de l'administration au patient de catécholamines. Aussi, dans le cas des patients en état de choc septique ou ayant présenté un choc septique, l'échantillon à tester provient, de préférence, d'un prélèvement obtenu dans les 6 jours ou à 6 jours (J6) après le début du choc septique, de préférence dans les 5 jours ou à 5 jours (J5) après le début du choc septique, de manière préférentielle dans les 4 jours ou à 4 jours (J4) après le début du choc septique, de manière encore préférée dans les 3 jours ou à 3 jours (J3) après le début du choc septique, ou encore dans les deux jours ou à 2 jours (J2) après le début du choc septique, ou encore dans les 24h ou à 24h (J1) après le début du choc septique. Autrement dit, l'échantillon à tester est, de préférence, issu d'un prélèvement effectué chez le patient pour lequel on souhaite évaluer un risque de complication, dans les 6 jours, dans les 5 jours, dans les 4 jours, dans les 3 jours, dans les 2 jours, ou dans les 24h, notamment à 6 jours, à 5 jours, à 4 jours, à 3 jours, à 2 jours ou à 24h, après l'admission en service de réanimation ou le début du choc septique, respectivement.

Dans le cas où le procédé selon l'invention est appliqué, non pas à un patient admis en service de réanimation, mais à un patient ayant subi une chirurgie, notamment une chirurgie lourde (du type cardiaque ou abdominale par exemple) ou transplantation, dont le suivi est assuré dans un service autre qu'un service de réanimation, les temps indiqués dans le cadre de la description de l'invention pour les prélèvements concernés seront calculés, non pas à partir de l'admission en service de réanimation, mais à partir du début de la chirurgie.

Dans le cadre de l'invention, il est également possible pour la comparaison réalisée à l'étape iii) d'utiliser un rapport entre la quantité de transcrit(s) déterminée pour un échantillon biologique dudit patient correspondant à un prélèvement à un instant t (notamment dans les 3 jours ou à 3 jours (J3)) sur la quantité de transcrit(s) déterminée pour un échantillon biologique dudit patient correspondant à un prélèvement à un instant t' (notamment dans les 24 heures ou à 24 heures (J1)).

Les échantillons, sur lequel le procédé de l'invention, sont mis en œuvre, encore nommés échantillon à tester, seront d'origine humaine.

L'échantillon à tester peut être un fluide biologique, par exemple choisi parmi le sang, le sang total notamment tel que collecté de la voie veineuse, c'est-à-dire contenant les cellules blanches et rouges, les plaquettes et le plasma, ou encore un échantillon de sérum ou de plasma, de même que des composants desdits fluides, comme les cellules mononuclées du sang périphérique ou PBMC (« Peripheral Blood Mononuclear Cells »), ou des vésicules excrétées telles que des cellules ou des corps apoptotiques, des vésicules excrétées, comprenant notamment des exosomes et des microvésicules. Les échantillons biologiques préférés, qu'ils s'agissent des échantillons biologiques à tester ou des échantillons utilisés pour déterminer la valeur de référence, sont, de préférence des échantillons de sang total ou de PBMC.

Les échantillons à partir desquels peuvent être déterminées les valeurs de référence, encore nommés « échantillons de référence », peuvent être de différentes natures, et notamment de nature biologique tel que mentionné ci-dessus s'agissant de l'échantillon à tester (fluides biologiques), ou non, notamment des échantillons synthétiques contenant une quantité calibrée en transcrit(s) sélectionné(s). Avantageusement, si ces échantillons de référence sont des échantillons biologiques, ils seront de même nature que celle de l'échantillon biologique à tester ou tout du moins d'une nature compatible pour constituer une référence quant à la détection et/ou la quantification du ou des transcrits du gène *IL7R* sélectionné(s). En particulier, dans le cas d'une mise en œuvre du procédé chez des sujets humains, un échantillon biologique de référence sera un échantillon biologique humain. On utilisera également, de préférence, des échantillons biologiques correspondant au même fluide biologique ou même(s) composant(s), par exemple des échantillons de sang total, à la fois pour l'échantillon à tester et pour l'échantillon de référence.

Toute méthode de détection et/ou de quantification de transcrit bien connue de l'homme du métier peut être utilisée pour la mise en œuvre de l'invention. En particulier, de telles méthodes utilisent un ou plusieurs partenaires de liaison pour le ou les transcrit(s) sélectionné(s).

On sait que, de façon générale, les résultats des tests de détection d'analytes dépendent en grande partie des caractéristiques du ou des partenaires de liaison utilisés. Ainsi, dans le cas de la détection d'ARN par hybridation avec des sondes nucléotidiques, les résultats dépendent en particulier des caractéristiques de taille, de composition et de pourcentage de complémentarité des sondes, et que ces caractéristiques influent sur les valeurs mesurées avec ces sondes. On conçoit donc qu'il n'est pas possible de donner des valeurs de référence précises et que la ou les valeurs de référence adaptées à chaque partenaire de liaison utilisé peuvent être déterminées dans chaque cas par de simples expériences de routine.

En particulier, la valeur de référence sera choisie, en fonction de la méthode utilisée pour déterminer la quantité dudit au moins un transcrit du gène *IL7R* et sera représentative de la population dont est issue le patient pour lequel on souhaite évaluer le risque.

L'homme du métier saura déterminer la valeur de référence avec laquelle la quantité dudit au moins un transcrit déterminée pour ledit échantillon biologique ou la valeur dérivée de cette quantité utilisée pour la comparaison doit être comparée, en fonction de la comparaison à effectuer. En particulier, un test statistique adapté pourra être utilisé pour déterminer cette valeur de référence. Par exemple, il est possible d'effectuer la comparaison entre des populations ou types d'échantillons différents, à partir de l'évolution dans le temps d'une même population ou du même type d'échantillon. Il faut bien comprendre que l'on appelle ici valeur de référence soit une valeur discrète, soit un intervalle de valeurs correspondant à une zone d'indétermination. Bien évidemment, lorsque la valeur mesurée est incluse dans l'intervalle d'indétermination, ou est très proche de la valeur de référence dans le cas d'une valeur discrète, on ne peut pas conclure définitivement et il convient de conduire des investigations supplémentaires.

Dans le cadre de l'invention, la valeur de référence peut être déterminée de différentes manières : en particulier, soit la valeur de référence est obtenue à partir d'un échantillon de référence issu du même patient et obtenu lors d'un prélèvement antérieur, soit la valeur de référence est obtenue à partir d'un échantillon de référence d'un individu de référence ou d'échantillons de référence d'une population de référence.

Une valeur de référence obtenue à partir d'un échantillon de référence issu du même patient obtenu lors d'un prélèvement antérieur sera nommée valeur de référence interne. Une valeur de référence obtenue à partir d'un échantillon de référence d'un individu de référence ou d'échantillons de référence d'une population de référence sera nommée valeur de référence externe.

Une valeur de référence interne peut correspondre à, ou être dérivée de, la quantité dudit au moins un transcrit du gène *IL7R* mesurée dans un échantillon biologique issu du même dit patient lors d'un prélèvement antérieur, c'est-à-dire dans un échantillon biologique issu du patient chez qui on souhaite évaluer le risque de complication et obtenu antérieurement par rapport à l'échantillon à tester. Par « antérieur » ou « antérieurement », on entend de manière plus précoce dans le temps.

De préférence, une valeur de référence interne est obtenue à partir d'un échantillon biologique qui est directement antérieur par rapport à l'échantillon à tester, c'est-à-dire celui qui précède l'échantillon à tester dans l'ordre des prélèvements effectués chez le patient.

Selon une mise en œuvre particulièrement avantageuse, l'échantillon de référence interne est issu d'un prélèvement obtenu dans les 2 jours ou dans le jour, ou encore à 2 jours ou à 1 jour après l'admission en réanimation du patient, ce qui permet de déterminer de manière très précoce le risque de complication du patient testé. Dans le cas où le patient est en état de choc septique ou a été en état de choc septique, ces délais seront calculés à compter du début du choc septique.

A titre d'exemple, le prélèvement antérieur est effectué dans les ou à 48h après l'admission en réanimation du patient et, de préférence, au moins 24h avant le prélèvement de l'échantillon à tester. De manière préférée, le prélèvement antérieur est effectué à 24h après l'admission en réanimation et, le prélèvement correspondant à l'échantillon à tester est effectué à 72h après l'admission en réanimation. Dans le cas d'un patient en état de choc septique ou ayant été en état de choc septique, le prélèvement antérieur sera, de préférence, effectué dans les ou à 48h après le début du choc septique et, de préférence, au moins 24h avant le prélèvement de l'échantillon à tester. De manière préférée, le prélèvement antérieur est effectué à 24h après le début du choc septique et le prélèvement correspondant à l'échantillon à tester est effectué à 72h après le début du choc septique.

De manière préférée, le procédé de l'invention permet de conclure à un risque de complication, et en particulier de mortalité chez le patient, lorsque la quantité dudit au moins un transcrit déterminée pour ledit échantillon biologique à tester ou une valeur dérivée de cette quantité qui est mesurée dans l'échantillon à tester n'est pas significativement augmentée par rapport à la valeur de référence interne. Il est à la portée de l'homme du métier de déterminer le pourcentage d'augmentation significatif qui dépendra, notamment, du type d'échantillon à tester (par exemple sang total, PBMC, sous-populations cellulaires), du type d'analyse, voire de l'appareil sur lequel l'analyse est effectuée, en fonction de s'il souhaite favoriser la spécificité dans le cas d'un test d'exclusion ou la sensibilité dans le cas d'un test d'inclusion, selon le traitement à appliquer ou encore la pathologie dont est atteint le patient.

Dans le cas maintenant de l'utilisation d'une valeur de référence externe, celle-ci peut correspondre à, ou être obtenue à partir de, la quantité dudit au moins un transcrit, mesurée dans un échantillon biologique issu d'un patient ayant subi une agression ou une infection générant un syndrome de réponse inflammatoire systémique dont on sait qu'il n'a pas connu de complication, notamment d'un patient présentant un sepsis dont on sait qu'il n'a pas connu de complication, et de préférence, d'un patient en choc septique dont on sait qu'il n'a pas connu de complication.

En particulier, lorsque le risque de complication à évaluer est un risque de mortalité, la valeur de référence externe peut correspondre à, ou être obtenue à partir de, la quantité dudit au moins un transcrit, mesurée dans un échantillon biologique de référence issu d'un patient ayant subi une agression ou une infection générant un syndrome de réponse inflammatoire systémique dont on sait qu'il a survécu, notamment d'un patient présentant un sepsis dont on sait qu'il a survécu, et de préférence, d'un patient en choc septique dont on sait qu'il a survécu.

Dans ce cas, la mesure de la quantité dudit au moins un transcrit déterminée qui sert pour la détermination de valeur de référence externe est de préférence effectuée en parallèle, c'est-à-dire en même temps que la mesure de la quantité dudit au moins un transcrit du gène *IL7R* qui est faite dans l'échantillon issu du patient chez qui on souhaite évaluer le risque de complication, bien que le prélèvement de l'échantillon biologique de référence ait été effectué antérieurement à celui de l'échantillon à tester.

La valeur de référence peut également correspondre à, ou être obtenue à partir d'une valeur moyenne de la quantité dudit au moins un transcrit qui est mesurée sur un pool d'échantillons issu de patients ayant subi une agression ou une infection générant un syndrome de réponse inflammatoire systémique (SIRS) dont on sait qu'ils n'ont pas connu de complication, notamment de patients présentant un sepsis dont on sait qu'ils n'ont pas connu de complication, et, de préférence, de patients en état de choc septique dont on sait qu'ils n'ont pas connu de complication.

En particulier, lorsque le risque de complication à évaluer est un risque de mortalité, la valeur de référence peut également correspondre à, ou être obtenue à partir d'une valeur moyenne de la quantité dudit au moins un transcrit qui est mesurée sur un pool d'échantillons issu de patients ayant subi une agression ou une infection générant un syndrome de réponse inflammatoire systémique (SIRS) dont on sait qu'ils ont survécu, notamment de patients présentant un sepsis dont on sait qu'ils ont survécu, et, de préférence, de patients en état de choc septique dont on sait qu'ils ont survécu.

Dans ce cas, la détermination de la valeur de référence externe est de préférence effectuée préalablement à la mesure de la quantité dudit au moins un transcrit du gène *IL7R* qui est faite dans l'échantillon issu du patient chez qui on cherche à évaluer le risque de mortalité, le prélèvement des échantillons de référence destinés à être « poolés » ayant été effectué antérieurement à celui de l'échantillon à tester.

En particulier, il est conclu à un risque accru de complication, et en particulier de mortalité chez ledit patient, lorsque la quantité dudit au moins un transcrit du gène *IL7R* dans l'échantillon biologique du patient est significativement diminuée par rapport à la valeur de référence externe. C'est en particulier le cas, quand le pronostic est basé sur la prise en compte de la quantité du transcrit IL7R-001, IL7R-002, IL7R-003, IL7R-005 ou IL7R-007, ou de la quantité globale de plusieurs transcrits, comprenant de préférence 2, 3, 4 ou tous les transcrits choisis parmi les transcrits IL7R-001, IL7R-002, IL7R-003, IL7R-005 et IL7R-007.

L'homme du métier sera à même de déterminer lorsqu'une diminution est jugée significative, en fonction notamment du type d'échantillons testés (par exemple sang total ou PBMC), du type de détection pratiquée (avec ou sans amplification), voire de l'appareil d'analyse utilisé, en fonction de s'il souhaite favoriser la spécificité dans le cas d'un test d'exclusion ou la sensibilité dans le cas d'un test d'inclusion, selon le traitement à appliquer ou encore la pathologie dont est atteint le patient.

En particulier, l'échantillon biologique à tester est issu d'un patient en état de choc septique au moment du prélèvement de l'échantillon biologique à tester ou ayant été en état de choc septique et la valeur de référence est dite externe et correspond à, ou est dérivée de, la quantité dudit au moins un transcrit du gène *IL7R* mesurée dans un échantillon biologique de référence issu d'un patient en état de choc septique au moment du prélèvement de l'échantillon de référence ou ayant été en état de choc septique, dont on sait qu'il n'a pas connu de complication, et en particulier dont on sait qu'il a survécu, ou bien correspond à une valeur moyenne de la quantité dudit au moins un transcrit du gène *IL7R* qui est mesurée sur un pool d'échantillons de référence issu de patients en état de choc septique au moment du prélèvement des échantillons de référence ou ayant été en état de choc septique, dont on sait qu'ils n'ont pas connu de complication, et en particulier dont on sait qu'ils ont survécu.

Pour obtenir une telle valeur de référence externe, le ou les échantillons de référence utilisés sont, de préférence, issus de personnes ayant les mêmes caractéristiques ou une majorité de caractéristiques communes, notamment du même sexe et/ou d'un âge similaire ou identique et/ou de même origine ethnique, avec celles du sujet ou patient chez qui on souhaite évaluer le risque de complication, et en particulier de mortalité. L'échantillon de référence peut également dans ce cas être constitué par tout échantillon, biologique ou non biologique, qui a été préalablement calibré pour contenir la quantité dudit au moins un transcrit du gène *IL7R* qui correspond à la quantité moyenne mesurée dans un pool d'échantillons de patients ayant subi une agression ou une infection générant un syndrome de réponse inflammatoire systémique (et en particulier de patients présentant un sepsis, et de préférence de patients en état de choc septique), dont on sait qu'ils n'ont pas connu de complication, et de préférence dont on sait qu'ils ont survécu, ou qui a été préalablement calibré pour contenir la quantité dudit au moins un transcrit du gène *IL7R* qui correspond à la quantité moyenne mesurée dans un pool d'échantillons de patients ayant subi une agression ou une infection générant un syndrome de réponse inflammatoire systémique (et en particulier de patients présentant un sepsis, et de préférence de patients en état de choc septique), dont on sait qu'ils n'ont pas connu de complication, et de préférence dont on sait qu'ils ont survécu.

De même, pour la détermination de la valeur de référence externe et la détection ou la quantification dudit au moins un transcrit du gène *IL7R* dans l'échantillon à tester, on utilisera de préférence des échantillons prélevés au même moment, notamment par rapport à l'admission en service de réanimation ou par rapport au début du choc septique selon le cas.

En particulier, l'échantillon biologique à tester est issu d'un patient en état de choc septique au moment du prélèvement de l'échantillon biologique à tester ou ayant été en état de choc septique et la mesure de la quantité dudit au moins un transcrit du gène *IL7R* est réalisée dans un échantillon à tester et, le cas échéant dans le ou les échantillons biologiques utilisés pour obtenir la valeur de référence externe, qui correspond(ent) à un prélèvement effectué dans les 6 jours après le début du choc septique, de préférence le 3^{ème} jour suivant le début du choc septique, et en particulier 72h après le début du choc septique.

Plus généralement, lorsque l'échantillon biologique à tester est issu d'un patient ayant subi une agression ou une infection générant un syndrome de réponse inflammatoire systémique qui a été admis en service de réanimation, la mesure de la quantité dudit au moins un transcrit du gène *IL7R* est réalisée dans un échantillon à tester et, le cas échéant dans le ou les échantillons biologiques utilisés pour obtenir la valeur de référence externe, issu(s) d'un prélèvement effectué, de préférence, dans les 6 jours après l'admission en réanimation du patient, de préférence le 3^{ème} jour suivant l'admission, et en particulier 72h après l'admission.

En particulier, pour un patient en état de choc septique au moment du prélèvement de l'échantillon biologique à tester ou ayant été en état de choc septique, à l'étape ii) des procédés selon l'invention, la comparaison pourra être effectuée directement à partir de la quantité dudit au moins un transcrit du gène *IL7R* mesurée, à la fois pour l'échantillon à tester et pour la détermination de la valeur de référence externe, dans un échantillon biologique provenant d'un prélèvement réalisé le 3^{ème} jour suivant le début du choc septique, et en particulier 72h après, ou à partir du rapport entre la quantité dudit au moins un transcrit du gène *IL7R* mesurée, à la fois pour l'échantillon à tester et pour la détermination de la valeur de référence externe, dans un échantillon biologique provenant d'un prélèvement réalisé le 3^{ème} jour suivant le début du choc septique, et en particulier 72h après, et la quantité, à la fois pour l'échantillon à tester et pour la détermination de la valeur de référence, dans un échantillon biologique provenant d'un prélèvement réalisé dans les 24h, et notamment à 24h suivant le choc septique. Dans ce cas, la comparaison est réalisée à l'étape iii) entre :
- le rapport de la quantité de transcrit(s) déterminée pour un échantillon biologique du patient dont on souhaite obtenir le diagnostic correspondant à un prélèvement à un instant **t** (notamment dans les 3 jours ou à 3 jours (J3) suivant le début du choc septique) sur la quantité de transcrit(s) déterminée pour un échantillon biologique dudit patient correspondant à un prélèvement à un instant **t'** (notamment dans les 24 heures ou à 24 heures (J1) suivant le début du choc septique) et
- le rapport de la quantité de transcrit(s) déterminée pour un échantillon biologique du patient ou de la population de référence correspondant à un prélèvement à un instant **t** (notamment dans les 3 jours ou à 3 jours (J3) suivant le début du choc septique) sur la quantité de transcrit(s) déterminée pour un échantillon biologique dudit patient ou population de référence correspondant à un prélèvement à un instant **t'** (notamment dans les 24 heures ou à 24 heures (J1) suivant le début du choc septique).

Plus généralement, lorsque l'échantillon biologique à tester est issu d'un patient ayant subi une agression ou une infection générant un syndrome de réponse inflammatoire systémique qui a été admis en service de réanimation, la comparaison est, de préférence, effectuée directement à partir de la quantité dudit au moins un transcrit du gène *IL7R* mesurée, pour l'échantillon à tester et pour la détermination de la valeur de référence externe, dans un échantillon biologique provenant d'un prélèvement réalisé le 3^{ème} jour suivant le début de l'admission du patient en service de réanimation (et en particulier 72h après) ou à partir du rapport entre la quantité dudit au moins un transcrit du gène *IL7R* mesurée, pour l'échantillon à tester et pour la détermination de la valeur de référence externe, dans un échantillon biologique provenant d'un prélèvement réalisé le 3^{ème} jour suivant l'admission (et en particulier 72h après) et la quantité mesurée, pour l'échantillon à tester et pour la détermination de la valeur de référence, dans un échantillon biologique provenant d'un prélèvement réalisé dans les 24h, et notamment à 24h suivant le choc septique.

Dans ce cas, la comparaison est réalisée à l'étape iii) entre :
- le rapport de la quantité de transcrit(s) déterminée pour un échantillon biologique du patient dont on souhaite obtenir le diagnostic correspondant à un prélèvement à un instant **t** (notamment dans les 3 jours ou à 3 jours (J3) suivant l'admission en réanimation) sur la quantité de transcrit(s) déterminée pour un échantillon biologique dudit patient correspondant à un prélèvement à un instant **t'** (notamment dans les 24 heures ou à 24 heures (J1) suivant l'admission en réanimation) et
- le rapport de la quantité de transcrit(s) déterminée pour un échantillon biologique du patient ou de la population de référence correspondant à un prélèvement à un instant **t** (notamment dans les 3 jours ou à 3 jours (J3) suivant le début du choc septique) sur la quantité de transcrit(s) déterminée pour un échantillon biologique dudit patient ou population de référence correspondant à un prélèvement à un instant **t'** (notamment dans les 24 heures ou à 24 heures (J1) suivant le début du choc septique).

Dans le cadre de l'invention, on entend, par détection d'un transcrit, la mise en évidence dudit transcrit lui-même dans l'échantillon biologique, par détection directe dudit transcrit, par tout procédé connu de l'homme du métier permettant de déterminer la présence dudit transcrit dans un échantillon, ou par détection indirecte du transcrit après transformation de ce dernier en ADN, ou après amplification dudit transcrit ou après amplification de l'ADN obtenu après transformation de dudit transcrit en ADN. Dans le cadre de l'invention, la détection s'accompagnera d'une quantification du ou des transcrits sélectionnés, c'est-à-dire que la concentration d'un transcrit ou de plusieurs transcrits, dans ce cas de manière globale ou individuelle, sera déterminée directement ou indirectement.

La détection directe d'un transcrit dans l'échantillon biologique peut être mise en œuvre par tout moyen connu de l'homme du métier, comme par exemple par hybridation avec un partenaire de liaison spécifique ou non du transcrit à détecter, le cas échéant après amplification par la technique PCR, avec ou sans sonde, ou NASBA, ou comme par exemple par séquençage (Cloonan *et al.,* 2008; Emrich *et al.,* 2007; Mortazavi *et al.,* 2008).

Par hybridation, on entend le processus au cours duquel, dans des conditions appropriées, deux fragments nucléotidiques se lient avec des liaisons hydrogènes stables et spécifiques pour former un complexe double brin.

Les partenaires de liaison d'un transcrit à détecter sont tout partenaire susceptible de se lier audit transcrit, et en particulier des partenaires de liaison spécifiques. A titre d'exemple de partenaires de liaison spécifiques, on peut citer les sondes d'hybridation et les amorces d'amplification, et toute autre molécule capable de se lier au transcrit à détecter. Il est possible d'utiliser des partenaires de liaison spécifique, c'est-à-dire se liant essentiellement, voire exclusivement, à un seul transcrit ou se liant à plusieurs transcrits du gène *IL7R,* comme illustré dans les exemples ci-après.

Par sonde d'hybridation, on entend un fragment nucléotidique comprenant de 5 à 100 motifs nucléiques, notamment de 10 à 35 motifs nucléiques, possédant une spécificité d'hybridation dans des conditions déterminées pour former un complexe d'hybridation avec un ou plusieurs transcrits du gène *IL7R.* La sonde d'hybridation peut comprendre un marqueur permettant sa détection et est alors nommée sonde de détection.

Au sens de la présente invention, on entend par amorce d'amplification, un fragment nucléotidique comprenant de 5 à 100 motifs nucléiques, préférentiellement de 15 à 30 motifs nucléiques permettant l'initiation d'une polymérisation enzymatique, telle que notamment une réaction d'amplification enzymatique. Par réaction d'amplification enzymatique, on entend un processus générant de multiples copies d'un fragment nucléotidique par l'action d'au moins une enzyme. De telles réactions d'amplification sont bien connues de l'homme du métier et on peut citer notamment les techniques suivantes :
- PCR (Polymerase Chain Reaction), telle que décrite dans les brevets US 4,683,195, US 4,683,202 et US 4,800,159,
- LCR (Ligase Chain Reaction), exposée par exemple dans la demande de brevet EP 0 201 184,
- RCR (Repair Chain Reaction), décrite dans la demande de brevet WO 90/01069,
- 3SR (Self Sustained Sequence Replication) avec la demande de brevet WO 90/06995,
- NASBA (Nucleic Acid Sequence-Based Amplification) avec la demande de brevet WO 91/02818,
- TMA (Transcription Mediated Amplification) avec le brevet US 5,399,491 et
- LAMP (Loop mediated isothermal amplification) avec le brevet US6410278.

Lorsque l'amplification enzymatique est une PCR, elle est réalisée après une réaction de transcription inverse, menée en une ou deux étapes, et est nommée classiquement RT-PCR (RT pour « reverse transcription »). Lors de la RT-PCR, le réactif spécifique par un ou plusieurs transcrits comprend au moins 2 amorces d'amplification, qui sont soit spécifiques du ou des transcrits cibles dans le cas d'une RT-PCR en une étape (EP 0569272), soit spécifiques du ou des ADN correspondant au(x) transcrit(s) cible(s) dans le cas d'une RT-PCR en deux étapes (Goblet *et al.,* 1989) .

Par détection, on entend soit une méthode physique, soit une méthode chimique avec un agent colorant intercalant tel que SYBR® Green I ou le bromure d'éthidium, soit une méthode de détection à l'aide d'un marqueur. De nombreuses méthodes de détection existent pour la détection des acides nucléiques (Keller G.H., 1993; Kricka, 1999).

Par marqueur, on entend un traceur capable d'engendrer un signal que l'on peut détecter. Une liste non limitative de ces traceurs comprend les enzymes qui produisent un signal détectable par exemple par colorimétrie, fluorescence ou luminescence, comme la peroxydase de raifort, la phosphatase alcaline, la beta-galactosidase, la glucose-6-phosphate déshydrogénase; les chromophores comme les composés fluorescents, luminescents ou colorants ; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme ³²P, ³⁵S ou ¹²⁵I.

Au sens de la présente invention, la sonde d'hybridation peut être une sonde dite de détection. Dans ce cas, la sonde dite de détection est marquée au moyen d'un marqueur tel que défini précédemment. Grâce à la présence de ce marqueur, on peut détecter la présence d'une réaction d'hybridation entre une sonde de détection donnée et le transcrit à détecter.

Concernant la PCR quantitative en temps réel (Real Time quantitative PCR), pour des applications de diagnostic deux types de marquage pour une hybridation spécifique sont généralement utilisés :
- Il est, tout d'abord, possible d'utiliser des méthodes utilisant une sonde entre deux amorces. En particulier, on pourra utiliser des sondes TaqMan®, telles que décrites par Espy *et al.,* 2006; Heid *et al.,* 1996; Holland *et al.,* 1991 ; des balises moléculaires également nommées Molecular beacons, telles que décrites par Espy *et al.,* 2006; Mhlanga and Malmberg, 2001; Sigma, 2008 ; des sondes d'hybridation adjacentes, dites HybProbes (FRET) ; ou encore des CPT (pour « Cycling probe technology »), telles que décrites par Duck *et al.,* 1990.
- Il est également possible d'utiliser des méthodes utilisant des amorces marquées. De telles amorces peuvent être des amorces scorpion ou Scorpion®, telles que décrites dans Sigma, 2008 ; des amorces Plexor, telles que décrites par Buh Gasparic *et al,* 2010 ; des amorces utilisées dans la technique AmpliFluor®, telle que décrite par Bio-Rad Laboratories, 2006; Nazarenko *et al.,* 1997 ; des amorces LUX (light upon extension), telles que décrites par Bio-Rad Laboratories, 2006; Buh Gasparic *et al.,* 2010; Nazarenko *et al.,* 2002 ; ou encore des amorces BD Qzyme ™ telles que décrites dans Bio-Rad Laboratories, 2006; Clontech, 2003.

La sonde d'hybridation peut être également une sonde dite de capture. Dans ce cas, la sonde dite de capture est immobilisée ou immobilisable sur un support solide par tout moyen approprié, c'est-à-dire directement ou indirectement, par exemple par covalence ou adsorption. Comme support solide, on peut utiliser des matériaux de synthèse ou des matériaux naturels, éventuellement modifiés chimiquement, notamment les polysaccharides tels que les matériaux à base de cellulose, par exemple du papier, des dérivés de cellulose tels que l'acétate de cellulose et la nitrocellulose ou le dextrane, des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques ; des gels etc. Le support solide peut être sous la forme d'une plaque de microtitration, d'une membrane comme décrit dans la demande WO-A-94/12670, d'une particule.

On peut également immobiliser sur le support plusieurs sondes de capture différentes, chacune étant spécifique d'un transcrit cible. En particulier, on peut utiliser comme support une biopuce sur laquelle peuvent être immobilisées un grand nombre de sondes. Par biopuce, on entend un support solide de dimension réduite où est fixée une multitude de sondes de capture à des positions prédéterminées. Le concept de biopuce, ou puce à ADN, date du début des années 90. Il repose sur une technologie pluridisciplinaire intégrant la micro-électronique, la chimie des acides nucléiques, l'analyse d'images et l'informatique. Le principe de fonctionnement repose sur un fondement de la biologie moléculaire: le phénomène d'hybridation, c'est-à-dire l'appariement par complémentarité des bases de deux séquences d'ADN et/ou d'ARN. La méthode des biopuces repose sur l'emploi de sondes de capture fixées sur un support solide sur lesquelles on fait agir un échantillon de fragments nucléotidiques cibles marqués directement ou indirectement avec des fluorochromes. Les sondes de capture sont positionnées de manière spécifique sur le support ou puce et chaque hybridation donne une information particulière, en relation avec le fragment nucléotidique cible. Les informations obtenues sont cumulatives, et permettent par exemple de quantifier un transcrit ou de plusieurs transcrits cibles. Après hybridation, le support ou puce est lavé(e), et les complexes transcrit / sondes de capture sont révélés par un ligand de forte affinité lié par exemple à un marqueur de type fluorochrome. La fluorescence est lue, par exemple, par un scanner et l'analyse de la fluorescence est traitée par informatique. On peut citer à titre indicatif, les puces à ADN mises au point par la société Affymetrix ("Accessing Genetic Information with High-Density DNA arrays") (Chee *et al.,* 1996; Pease *et al.,* 1994), pour les diagnostics moléculaires. Dans cette technologie, les sondes de capture sont généralement de tailles réduites, autour de 25 nucléotides. D'autres exemples de biopuces sont donnés dans de nombreuses publications (Cheng *et al.,* 1998, 1996; Ginot, 1997; Livache *et al.,* 1994; Ramsay, 1998) ou dans les brevets US-A-4,981,783, US-A-5,700,637, US-A-5,445,934, US-A-5,744,305 et US-A-5,807,522. La caractéristique principale du support solide doit être de conserver les caractéristiques d'hybridation des sondes de capture sur les fragments nucléotidiques cibles tout en générant un bruit de fond minimum pour la méthode de détection.

Les techniques d'immobilisation de sondes sur un support sont bien connues de l'homme du métier, comme par exemple le dépôt de sondes pré-synthétisées par impression ou microdéposition (demandes de brevet WO-A-00/71750, FR 00/14896, FR00/14691), ou encore la synthèse *in situ* (demandes de brevet WO 89/10977 et WO 90/03382).

Pour détecter le transcrit de l'échantillon biologique, une étape d'extraction peut être nécessaire. L'extraction est mise en œuvre par tous les protocoles d'extraction et de purification d'acides nucléiques bien connus de l'homme du métier. A titre indicatif, l'extraction d'acides nucléiques peut être réalisée par :
▪une étape de lyse des cellules présentes dans l'échantillon biologique, afin de libérer les acides nucléiques contenus dans les cellules du patient. A titre d'exemple, on peut utiliser les méthodes de lyse telles que décrites dans les demandes de brevet:
   - WO 00/05338 sur la lyse mixte magnétique et mécanique,
   - WO 99/53304 sur la lyse électrique,
   - WO 99/15321 sur la lyse mécanique.
      L'homme du métier pourra utiliser d'autres méthodes de lyse bien connues, telles que les chocs thermiques ou osmotiques ou les lyses chimiques par des agents chaotropiques tels que les sels de guanidium (US 5,234,809).
▪une étape de purification, permettant la séparation entre les acides nucléiques et les autres constituants cellulaires relargués dans l'étape de lyse. Cette étape permet généralement de concentrer les acides nucléiques, et peut être adapté à la purification d'ARN. A titre d'exemple, on peut utiliser des particules magnétiques éventuellement revêtues d'oligonucléotides, par adsorption ou covalence (voir à ce sujet les brevets US 4,672,040 et US 5,750,338), et ainsi purifier les acides nucléiques qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet: WO-A-97/45202 et WO-A-99/35500. On peut également utiliser de la silice, soit sous forme de colonne, soit sous forme de particules inertes (Boom *et al.,* 1990) ou magnétiques (Merck: MagPrep® Silica, Promega: MagneSil® Paramagnetic particles). D'autres méthodes très répandues reposent sur des résines échangeuses d'ions en colonne ou en format particulaire paramagnétique (Whatman: DEAE-Magarose) (Levison *et al.,* 1998). Une autre méthode est celle de l'adsorption sur support d'oxyde métallique (société Xtrana: matrice Xtra-Bind®).

Lorsque l'on souhaite extraire spécifiquement les ARN d'un échantillon biologique, on peut notamment réaliser une extraction par du phénol, du chloroforme et de l'alcool pour éliminer les protéines et précipiter les ARN avec de l'éthanol 100%. Les ARN peuvent alors être culotés par centrifugation, lavés et remis en solution.

De telles méthodes de détection et de quantification permettront de déterminer la quantité d'un ou plusieurs transcrits présents dans l'échantillon testé ou d'en donner une valeur dérivée. Une valeur dérivée de la quantité peut par exemple être la concentration absolue, calculée grâce à une courbe de calibration obtenue à partir de dilutions successives d'une solution d'amplicon de concentration connue. Elle peut également correspondre à la valeur de la quantité normalisée et calibrée, comme le CNRQ (Calibrated Normalized Relative Quantity, (Hellemans *et al.,* 2007)), qui intègre les valeurs d'un échantillon référence, d'un calibrateur et d'un ou plusieurs gènes de référence. A titre d'exemples de gène de référence, on peut citer les gènes PPIB, PPIA, GLYR1, RANBP3, HPRT1, 18S, GAPDH, RPLPO et ACTB.

La détermination de la quantité de plusieurs transcrits peut être mise en œuvre séquentiellement ou simultanément, selon les procédés classiquement connus de l'homme du métier, comme indiqué précédemment.

Dans le cadre de l'invention, la quantité dudit au moins un transcrit du gène *IL7R* sera, de préférence, mesurée par RT-PCR quantitative.

En particulier, la quantité dudit au moins un transcrit du gène *IL7R* sera mesurée avec au moins l'un des couples d'amorces d'amplification suivants, avec ou sans la sonde mentionnée :
- amorce sens de **SEQ ID N°12** et amorce anti-sens de **SEQ ID N°13,** et éventuellement sonde de **SEQ ID N°14,** qui permettent de détecter les transcrits IL7R-001, IL7R-002, IL7R-003, IL7R-005 et IL7R-007 et leurs variants,
- amorce sens de **SEQ ID N°15** et amorce anti-sens de **SEQ ID N°16,** et éventuellement sonde de **SEQ ID N°17,** qui permet de détecter le transcrit IL7R-001 et ses variants,
- amorce sens de **SEQ ID N°18** et amorce anti-sens de **SEQ ID N°19,** et éventuellement sonde de **SEQ ID N°20,** qui permet de détecter le transcrit IL7R-001 et ses variants,

Toutes les indications et préférences mentionnées ci-dessus s'agissant de la détection ou de la quantification du ou des transcrits du gène *IL7R* sélectionné(s) s'appliquent indifféremment que ce soit pour la détection ou la quantification dans l'échantillon à tester ou dans l'échantillon de référence.

Pour la mise en oeuvre du procédé de l'invention, l'invention a pour autre objet un kit de diagnostic comprenant les outils et/ou réactifs nécessaires à la détection d'au moins un transcrit du gène *IL7R.*

A titre non limitatif de réactifs nécessaires à la détection d'un ou plusieurs transcrits du gène *IL7R,* on peut citer les partenaires de liaison du ou desdits transcrits, tels que des sondes d'hybridation ou des amorces d'amplification.

En particulier, l'invention concerne les kits pour la mesure, *in vitro* ou *ex vivo,* de la quantité d'au moins un transcrit du gène *IL7R* dans un échantillon biologique, comprenant :
- des outils ou réactifs spécifiques permettant de mesurer la quantité dudit au moins un transcrit du gène *IL7R dans* ledit échantillon biologique, et
- un échantillon contrôle qui est un échantillon calibré pour contenir la quantité dudit au moins un transcrit du gène *IL7R* qui correspond à la quantité moyenne mesurée dans un pool d'échantillons de patients ayant subi une agression ou une infection générant un syndrome de réponse inflammatoire systémique, dont on sait qu'ils n'ont pas connu de complication, et de préférence dont on sait qu'ils ont survécu, et/ou un échantillon contrôle qui est un échantillon calibré pour contenir la quantité dudit au moins un transcrit du gène *IL7R* qui correspond à la quantité moyenne mesurée dans un pool d'échantillons de patients ayant subi une agression ou une infection générant un syndrome de réponse inflammatoire systémique, dont on sait qu'ils ont connu une complication, et de préférence dont on sait qu'ils n'ont pas survécu.

En particulier, un tel kit contient, en tant que réactifs spécifiques permettant de mesurer la quantité dudit au moins un transcrit du gène *IL7R* dans ledit échantillon biologique, au moins l'un des couples d'amorces d'amplification suivants, avec ou sans la sonde mentionnée :
- amorce sens de **SEQ ID N°12** et amorce anti-sens de **SEQ ID N°13,** et éventuellement sonde de **SEQ ID N°14,**
- amorce sens de **SEQ ID N°15** et amorce anti-sens de **SEQ ID N°16,** et éventuellement sonde de **SEQ ID N°17,**
- amorce sens de **SEQ ID N°18** et amorce anti-sens de **SEQ ID N°19,** et éventuellement sonde de **SEQ ID N°20.**

Un échantillon contrôle peut être un échantillon contenant une concentration donnée en transcrit(s) cible(s) ou en ADN complémentaire correspondant, qui peut être soit un échantillon synthétique contenant une concentration calibrée en transcrit(s) cible(s) ou en ADN complémentaire correspondant ou un échantillon biologique. Un échantillon contrôle peut notamment être un échantillon biologique issu d'au moins un patient ayant subi une agression ou une infection générant un syndrome de réponse inflammatoire systémique, dont on sait qu'il n'a pas connu de complication, et notamment qu'il a survécu, ou encore un échantillon biologique issu d'au moins un patient ayant subi une agression ou une infection générant un syndrome de réponse inflammatoire systémique dont on sait qu'il a connu une complication, et notamment qu'il n'a pas survécu. Ce type d'échantillon contrôle est notamment issu d'un ou de plusieurs patient(s) ayant subi une agression, telle que chirurgie, brûlure, traumatisme..., ou une infection générant un syndrome de réponse inflammatoire systémique (SIRS), notamment d'un ou de plusieurs patient(s) présentant un sepsis, et de préférence d'un ou de plusieurs patient(s) en état de choc septique.

L'invention couvre également l'utilisation d'un kit selon l'invention pour réaliser le procédé de l'invention, et en particulier pour évaluer le risque de complication, et en particulier de mortalité d'un patient ayant subi une agression, telle que chirurgie, brûlure, traumatisme..., ou une infection générant un syndrome de réponse inflammatoire systémique (SIRS), en particulier chez un patient présentant un sepsis, notamment un sepsis sévère. De préférence, l'utilisation du kit selon l'invention permet d'évaluer le risque de mortalité chez un patient qui est en état de choc septique.

La présente invention a également pour objet l'utilisation de la mesure, *in vitro* ou *ex vivo,* de la quantité d'au moins un transcrit du gène *IL7R,* d'au moins un transcrit du gène *IL7R* correspondant à un ARNm, dans un échantillon biologique d'un patient ayant subi une agression ou une infection générant un syndrome de réponse inflammatoire systémique, pour évaluer le risque de complication, et en particulier de mortalité, chez ledit patient. En particulier, ledit patient est en état septique, notamment sévère ou a subi une chirurgie, une brûlure ou un traumatisme générant un syndrome de réponse inflammatoire systémique. Selon des modes de réalisations particuliers, l'échantillon biologique à tester est issu d'un patient en état de choc septique ou ayant été en état de choc septique dans les 6 jours précédant le prélèvement de l'échantillon biologique à tester. De préférence, ledit au moins un transcrit du gène *IL7R* est choisi parmi les transcrits IL7R-001 de **SEQ ID N°2,** IL7R-002 de **SEQ ID N°3,** IL7R-003 de **SEQ ID N°4,** IL7R-005 de **SEQ ID N°6** et IL7R-007 de **SEQ ID N°8** et leurs variants, la séquence d'un variant présentant au moins 99% d'identité avec l'une desdites séquences. En particulier, ledit au moins un transcrit du gène *IL7R* est choisi parmi les transcrits comprenant au moins une partie du domaine transmembranaire, voire tout le domaine transmembranaire, de CD127, et correspond, de préférence au transcrit IL7R-001 de **SEQ ID N°2** ou à un de ses variants présentant au moins 99% d'identité avec ladite séquence.

Plus largement, tous les modes de réalisation préférés qui sont mentionnés ci-dessus concernant le procédé et leurs combinaisons constituent également des modes de réalisation préférés s'agissant de l'utilisation. L'utilisation selon l'invention pourra également comprendre la détection et/ou la quantification du ou des transcrits du gène *IL7R* sélectionné(s), combinée à l'estimation de l'un au moins des scores de sévérité SOFA et/ou SAPSII pour évaluer le risque de complication du patient ayant subi une agression ou une infection générant une réponse inflammatoire systémique (SIRS), et en particulier d'un patient qui est en état de choc septique. Dans ce mode de réalisation, le score SOFA est de préférence calculé, comme décrit par Vincent *et al.,* 1996, et/ou le score SAPSII est, de préférence calculé, comme décrit par Le Gall *et al.,* 1993.

Tous les modes de réalisation préférés qui sont mentionnés ci-dessus concernant le procédé et leurs combinaisons constituent également des modes de réalisation préférés, s'agissant du kit selon l'invention et de son utilisation et de l'utilisation de la mesure, *in vitro* ou *ex vivo,* de la quantité d'au moins un transcrit du gène *IL7R.*

Diverses autres caractéristiques ressortent des exemples ci-après en référence aux figures annexées qui montrent, à titre d'exemples non limitatifs, des formes de réalisation de l'objet de l'invention :
- la **Figure 1** est une représentation schématique de l'épissage de l'ARN messager du gène *IL7R,*
- la **Figure 2** représente les niveaux d'expression de différents transcrits du gène *IL7R* chez des volontaires sains (n=19) et chez des patients en choc septique à J1 et J3 après le début du choc septique (n=30). PCR-A : Transcrits IL7R-001, IL7R-002, IL7R-003, IL7R-005, IL7R-007 ; PCR-B : Transcrit IL7R-001; PCR-C : Transcrit IL7R-007. Le test de Mann-Whitney a été utilisé pour les comparaisons entre sujets sains et patients en choc septique à J1 ou à J3. Le test de Wilcoxon apparié a été utilisé pour les comparaisons entre J1 et J3 chez les patients en choc septique.

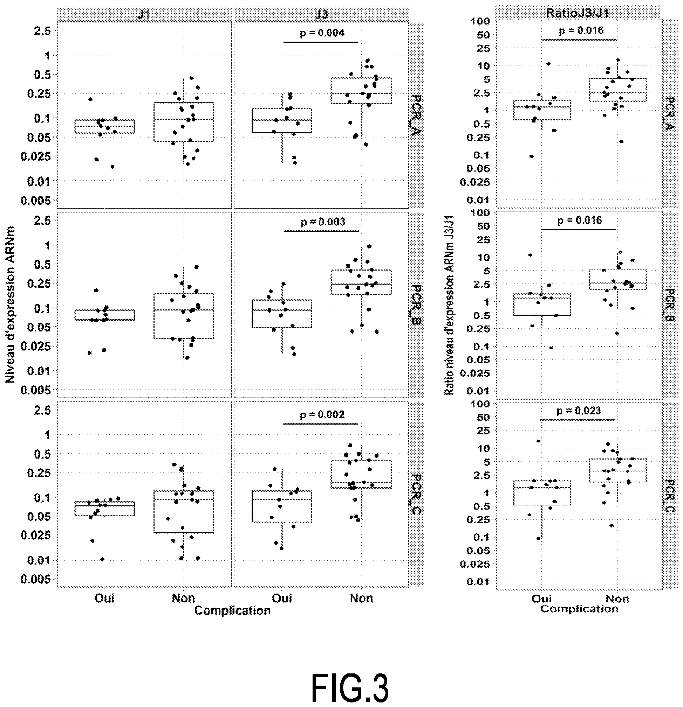

- la **Figure 3** représente les niveaux d'expression de différents transcrits du gène *IL7R* en fonction de la survenue ou non de complications chez des patients en choc septique, à J1, J3 ou pour le ratio J3/J1. Les niveaux d'expression sont exprimés en « Calibrated Normalized Relative Quantity » (CNRQ) avec HPRT1, comme gène de référence et ont été comparés par le test de Mann-Whitney. PCR-A : Transcrits IL7R-001, IL7R-002, IL7R-003, IL7R-005, IL7R-007 ; PCR-B : Transcrit IL7R-001; PCR-C : Transcrit IL7R-007.

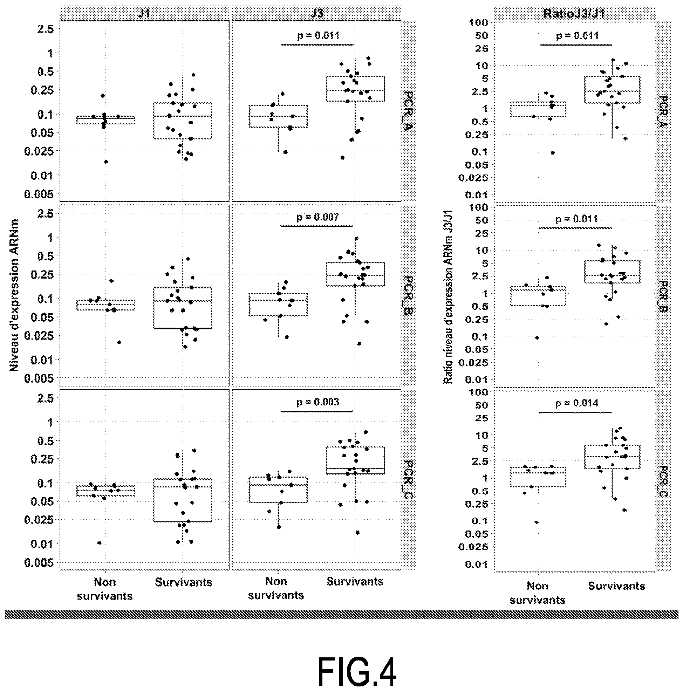

- la **Figure 4** représente les niveaux d'expression de différents transcrits du gène *IL7R* en fonction de la survenue ou non du décès à J28 chez des patients en choc septique, à J1, J3 ou pour le ratio J3/J1. Les niveaux d'expression sont exprimés en CNRQ avec HPRT1, comme gène de référence et ont été comparés par le test de Mann-Whitney. PCR-A : Transcrits IL7R-001, IL7R-002, IL7R-003, IL7R-005, IL7R-007 ; PCR-B : Transcrit IL7R-001; PCR-C : Transcrit IL7R-007.

### METHODES

### Patients et Echantillons biologiques

Des échantillons de sang total ont été prélevés sur des tubes PAXgene (PreAnalytix) sur 30 patients en choc septique aux jours 1 (J1) et 3 (J3) après le début du choc septique, puis ont été stockés (cohorte rétrospective).

A 28 jours après admission en réanimation pour choc septique, 9 patients n'ont pas survécu (« NS ») soit 30 %, alors que 21 patients ont survécu (« S ») sur les 30 patients.

Durant leur séjour en réanimation, 4 patients ont contracté une infection nosocomiale, soit 13%, alors que 26 patients sur les 30 patients n'en ont pas contracté.

Au total, 11 patients ont souffert de complications (décès à J28 ou survenue d'une infection nosocomiale en réanimation) soit 37%, alors que pour 19 patients sur les 30 patients, aucune complication n'est survenue.

Des échantillons de sang total, traités de la même façon que les échantillons des patients en choc septique, ont également été prélevés chez 19 sujets sains volontaires.

### Technique de détection

### Extraction de l'ARN et transcription inverse

L'extraction de l'ARN a été effectuée à l'aide du kit PAXgene Blood RNA (PreAnalytix) en suivant les recommandations du fabricant. La qualité des ARN extraits a été contrôlée sur Bioanalyseur (Agilent Technologies, Santa Clara, CA), en utilisant des puces RNA 6000 Nano. Avant l'étape d'élution de l'ARN, l'ADN génomique résiduel a été éliminé par l'action d'une DNAse. La qualité de l'ARN a ensuite été contrôlée à l'aide du kit RNA 6000 Nano sur un Bioanalyseur (Agilent Technologies), les prélèvements avec un RIN (RNA Integrity Number) supérieur à 6 étant considérés de bonne qualité. Enfin, la concentration d'ARN a été déterminée par fluorimétrie (kit RNA assay sur Qubit, Life Technologies).

L'ARN total (200 ng) a ensuite subi une transcription inverse en ADN complémentaire en utilisant le kit SuperScript® VILO™ cDNA Synthesis (Life Technologies, Chicago, IL). La solution d'ADNc ainsi obtenue a été diluée au 1/20 et stockée à -80°C jusqu'à la réaction de PCR quantitative.

### Technique de PCR quantitative

Les réactions de PCR ont été réalisées sur un LightCycler 480 (Roche Molecular Biochemicals, Bâle, Suisse) avec le kit Taqman Fast Advanced Master Mix PCR (Roche), dans un volume final de 20 µL contenant 0,5 M d'amorces et 0,1 M de sonde. Les PCR ont été réalisées avec une étape de dénaturation initiale de 10 min à 95°C, suivie de 45 cycles d'amplification en « touchdown PCR » (10 sec à 95°C, 29 sec à 68°C pour le premier cycle, avec une diminution de 0,5°C à chaque cycle jusqu'à atteindre 58°C, et extension de 1 s à 72°C). La méthode du maximum de la dérivée seconde a été utilisée *via* le logiciel du LightCycler pour déterminer de façon automatique le Cp (« crossing point ») pour chaque échantillon. Des courbes de calibration ont été générées en réalisant, pour 8 dilutions en série au 1/10 d'une solution mère d'amplicon standard de concentration connue. Les couples d'amorces et sondes utilisés sont listés dans le **Tableau 2 :**

**Tableau 2. Séquences des amorces et sondes des PCR pour la détection de différents transcrits du gène IL7R**

| | | **Séquences** | **Transcrits ciblés** |
|---|---|---|---|
| | | | IL7R-001 (NM_002185) |
| **PCR-A** | Amorce sens | **SEQ ID N°12** | IL7R-002 (ENST00000514217) |
| **(« plusieurs** | Amorce anti-sens | **SEQ ID N°13** | IL7R-003 (ENST00000506850) |
| **transcrits »)** | Sonde | **SEQ ID N°14** | IL7R-005 (ENST00000511031) |
| | | | IL7R-007 (ENST00000511982) |
| **PCR-B (Forme membranaire)** | Amorce sens | **SEQ ID N°15** | IL7R-001 (NM_002185) |
| | Amorce anti-sens | **SEQ ID N°16** | |
| | Sonde | **SEQ ID N°17** | |
| **PCR-C (Forme soluble potentielle)** | Amorce sens | **SEQ ID N°18** | IL7R-007 (ENST00000511982) |
| | Amorce anti-sens | **SEQ ID N°19** | |
| | Sonde | **SEQ ID N°20** | |

Les niveaux d'expression ont été exprimés en CNRQ (« Calibrated Normalized Relative Quantity »), d'après Hellemans *et al.,* 2007, en incluant un gène de référence et un calibrateur. Le gène de référence utilisé est HPRT1 (hypoxanthine phosphoribosyltransferase 1, NM_000194), mesuré par PCR (Amorce sens : CCAAAGATGGTCAAGGTCGC, amorce anti-sens : GACACAAACATGATTCAAATCC, sonde CAAGTTTGTTGTAGGATATGCCC). Le calibrateur était composé d'un pool d'ARN de volontaires sains. Ce calibrateur a suivi le même processus que les échantillons cliniques à partir de l'étape de transcription inverse.

### Analyses statistiques

Les analyses statistiques ont été réalisées grâce au logiciel RStudio (version 0.98.501). Les différences observées ont été considérées comme significatives pour des valeurs de p inférieures à 0,05.

### Analyse descriptive des niveaux d'expression des transcrits du gène IL7R

Les comparaisons des niveaux d'expression des transcrits du gène *IL7R* ont été réalisées par le test de Mann-Whitney, sauf dans le cas des comparaisons entre J1 et J3 chez les patients en choc septique, réalisées par le test de Wilcoxon apparié.

### Analyses de la capacité à prédire la survenue de complications ou du décès à J28

Les courbes ROC (*Receiver Operating Curves*) ont été générées et les aires sous la courbe, ainsi que leurs intervalles de confiance ont été calculés.

### RESULTATS

### Détection des transcrits du gène IL7R chez les sujets sains et les patients en choc septique

Le niveau d'expression des transcrits du gène *IL7R* a été mesuré, comme décrit ci-dessus dans les échantillons de sang total de 30 patients en choc septique et de 19 sujets sains volontaires. Les résultats sont présentés sur la **Figure 2****.**

L'ensemble des transcrits du gène *IL7R* est détecté à la fois chez les sujets sains et chez les patients en choc septique, à J1 et à J3 après le début du choc septique.

Le niveau d'expression du transcrit codant pour la forme membranaire de CD127 (Transcrit IL7R-001) est majoritaire par rapport au transcrit correspondant à une forme soluble potentielle (Transcrit IL7R-007), chez les sujets sains comme chez les patients en choc septique.

Les niveaux d'expression de l'ensemble des transcrits mesurés avec la PCR-A sont inférieurs chez les patients en choc septique à J1 et à J3 en comparaison avec les volontaires sains, et ce, également pour le transcrit codant pour la forme membranaire de CD127 (Transcrit IL7R-001 - PCR-B) et pour le transcrit correspondant à une forme soluble potentielle de CD127 (Transcrit IL7R-007 - PCR-C).

Chez les patients en choc septique, on observe une augmentation significative du niveau d'expression des différents transcrits du gène *IL7R* codant pour CD127 entre J1 et J3.

### Comparaison des niveaux d'expression des transcrits du gène IL7R codant pour CD127 en fonction de la survenue de complication chez les patients en choc septique

Comme le montre la **Figure 3****,** les niveaux d'expression des différents transcrits du gène *IL7R,* y compris celui codant pour la forme membranaire (Transcrit IL7R-001) et celui correspondant à une forme soluble potentielle (Transcrit IL7R-007), sont significativement plus faibles à J3 chez les patients qui souffriront de complications (décès ou infection nosocomiale constatée qui correspond à « oui » sur la **Figure 3**). Cette observation est également vraie pour le rapport des niveaux d'expression J3/J1.

### Capacité des transcrits du gène IL7R à prédire le risque de complication chez les patients en choc septique

La capacité prédictive de la mesure des niveaux d'expression de différents transcrits du gène *IL7R* a été étudiée au regard de l'événement à étudier, à savoir la survenue de complications. Les résultats sont représentés dans le **Tableau 3,** qui regroupe les aires sous la courbe de ROC (*Receiving Operating Curve*) ou AUC *(Area Under Curve*), ainsi que leurs intervalles de confiance à 95%.

**Tableau 3. Aires sous la courbe pour la prédiction du risque de complication pour les différents transcrits du gène IL7R**

| Transcrits | Jour | AUC | IC 95% |
|---|---|---|---|
| Tous transcrits (PCR-A) | J1 | 0,632 | [0,425-0,840] |
| | J3 | **0,813** | [0,657-0,970] |
| | Ratio J3/J1 | **0,766** | [0,571-0,960] |
| Transcrit membranaire IL7R-001 (PCR-B) | J1 | 0,627 | [0,419-0,832] |
| | J3 | **0,823** | [0,671-0,975] |
| | Ratio J3/J1 | **0,766** | [0,570-0,961] |
| Transcrit soluble IL7R-007 (PCR-C) | J1 | 0,665 | [0,464-0,866] |
| | J3 | **0,837** | [0,689-0,986] |
| | Ratio J3/J1 | **0,751** | [0,549-0,953] |

Les niveaux d'expression de différents transcrits du gène *IL7R* mesurés à J3, ou le ratio d'expression J3/J1, permettent donc de discriminer les patients qui souffriront de complications, de ceux qui n'en souffriront pas, avec des aires sous la courbe supérieures à 0,8 pour J3 et supérieures à 0,75 pour le ratio d'expression J3/J1.

### Comparaison des niveaux d'expression des transcrits du gène IL7R en fonction de la survenue du décès chez les patients en choc septique

Comme le montre la **Figure 4****,** les niveaux d'expression des différents transcrits du gène *IL7R,* y compris celui codant pour la forme membranaire (Transcrit IL7R-001) et celui correspondant à une forme soluble potentielle (Transcrit IL7R-007), sont significativement plus faibles à J3 chez les patients qui vont décéder (notés « Non survivants » sur la **Figure 4****)** dans les 28 jours suivant le choc septique, en comparaison avec les patients qui vont survivre (notés « Survivants » sur la **Figure 4****)** plus de 28 jours. Cette observation est également vraie pour le rapport des niveaux d'expression J3/J1.

### Capacité des transcrits à prédire le risque de décès chez les patients en choc septique

La capacité prédictive de la mesure des niveaux d'expression de différents transcrits du gène *IL7R* a été étudiée au regard de la survenue du décès dans les 28 jours suivant le début du choc septique. Les résultats sont représentés dans le **Tableau 4,** qui regroupe les aires sous la courbe de ROC et leurs intervalles de confiance à 95%.

**Tableau 4. Aires sous la courbe pour la prédiction de la survenue du décès pour les différents transcrits du gène IL7R**

| Transcrits | | Jour | AUC | IC 95% |
|---|---|---|---|---|
| Tous transcrits (PCR-A) | | J1 | 0,545 | [0,327-0,763] |
| | | J3 | **0,794** | [0,629-0,958] |
| | | Ratio J3/J1 | **0,794** | [0,633-0,954] |
| Transcrit membranaire (PCR-B) | IL7R-001 | J1 | 0,550 | [0,335-0,765] |
| | | J3 | **0,809** | [0,653-0,966] |
| | | Ratio J3/J1 | **0,794** | [0,631-0,956] |
| Transcrit soluble IL7R-007 | (PCR-C) | J1 | 0,603 | [0,394-0,812] |
| | | J3 | **0,836** | [0,692-0,980] |
| | | Ratio J3/J1 | **0,783** | [0,619-0,947] |

Les niveaux d'expression de différents transcrits du gène *IL7R* mesurés à J3, ou le ratio d'expression J3/J1, permettent donc de discriminer les patients qui vont décéder de ceux qui vont survivre, avec des aires sous la courbe supérieures à 0,75.

Cette étude montre que les niveaux d'expression de différents transcrits du gène *IL7R* permettent d'identifier les patients les plus à risque de souffrir de complications après un choc septique. Plus précisément, à J3, les patients chez qui des complications vont survenir, présentent des niveaux d'expression des transcrits du gène *IL7R* plus faibles que les patients qui ne vont souffrir d'aucune complication. L'évolution des niveaux d'expression des différents transcrits du gène *IL7R* entre J1 et J3 est également informative, les niveaux d'expression restant stables chez les patients qui vont souffrir de complications, alors qu'ils augmentent chez les patients qui ne vont souffrir d'aucune complication. Alors qu'au niveau protéique, seule la forme soluble était associée à la survenue d'un mauvais pronostic (Venet *et al.,* 2012), les différents transcrits du gène *IL7R,* qui correspondent à la forme membranaire ou à des formes solubles, et qui sont dosés simultanément ou individuellement, sont capables d'identifier les patients les plus à risque de survenue de complications.

### Références

Bio-Rad Laboratories, 2006, Real-Time PCR: Applications Guide.
Bone, R.C., et al., 1992, Chest 101, 1644-1655.
Boom, R., et al., 1990, J. Clin. Microbiol. 28, 495-503.
Buh Gasparic, M., et al., 2010, Anal. Bioanal. Chem. 396, 2023-2029.
Carini, C., et al., 1994, Eur. J. Immunol. 24, 2927-2934.
Chee, M., et al., 1996, Science 274, 610-614.
Cheng, J., et al., 1998, Nat. Biotechnol. 16, 541-546.
Cheng, J., et al. 1996, Mol. Diagn. J. Devoted Underst. Hum. Dis. Clin. Appl. Mol. Biol. 1, 183-200.
Clontech, 2003, BD QZyme Assays for Quantitative PCR.
Cloonan, N., et al., 2008, Nat. Methods 5, 613-619.
Duck, P., et al., 1990, BioTechniques 9, 142-148.
Emrich, S.J., et al., 2007, Genome Res. 17, 69-73.
Espy, M.J., et al., 2006, Clin. Microbiol. Rev. 19, 165-256.
Ginot, F., 1997, Hum. Mutat. 10, 1-10.
Goblet, C., et al., 1989, Nucleic Acids Res. 17, 2144.
Goodwin, R.G., et al., 1990, Cell 60, 941-951.
Heid, C.A., et al., 1996, Genome Res. 6, 986-994.
Hellemans, J., et al., 2007, Genome Biol. 8, R19.
Holland, P.M., et al., 1991, Proc. Natl. Acad. Sci. U. S. A. 88, 7276-7280.
Jiang, Q., et al., 2005, Cytokine Growth Factor Rev. 16, 513-533.
Keller G.H., et al., 1993), Stockton Press.
Kricka, L.J., 1999, Clin. Chem. 45, 453-458.
Le Gall, J.R., et al., 1993, J. Am. Med. Assoc. 270, 2957-2963.
Levison, P.R., et al., 1998, J. Chromatogr. A 827, 337-344.
Livache, T., et al., 1994, Nucleic Acids Res. 22, 2915-2921.
Mhlanga, M.M., and Malmberg, L., 2001, Methods San Diego Calif 25, 463-471.
Mortazavi, A., et al., 2008, Nat. Methods 5, 621-628.
Nazarenko, I., et al., 2002, Nucleic Acids Res. 30, e37.
Nazarenko, I.A., et al., 1997, Nucleic Acids Res. 25, 2516-2521.
Park, L.S., et al., 1990, J. Exp. Med. 171, 1073-1089.
Pease, A.C., et al., 1994, Proc. Natl. Acad. Sci. U. S. A. 91, 5022-5026.
Ramsay, G., 1998), Nat. Biotechnol. 16, 40-44.
Rose, T., et al., 2009, J. Immunol. 182, 7389-7397.
Sigma, 2008, qPCR Technical Guide.
Venet, F., et al., 2012, J. Immunol. 189, 5073-5081.
Vincent, J.L., et al., 1996, Intensive Care Med. 22, 707-710.
Vranjkovic, A., et al., 2007, Int. Immunol. 19, 1329-1339.

**SEQ ID N°12** : amorce sens PCR-A (transcrits IL7R-001, IL7R-002, IL7R-003, IL7R-005 et IL7R-007)
   GGAAGTGAATGGATCGCAGC
**SEQ ID N°13 :** amorce anti-sens PCR-A (transcrits IL7R-001, IL7R-002, IL7R-003, IL7R-005 et IL7R-007)
   GGCACTTTACCTCCACGAG
**SEQ ID N°14** : sonde PCR-A (transcrits IL7R-001, IL7R-002, IL7R-003, IL7R-005 et IL7R-007)
   CTGTGCTTTTGAGGACCCAGAT
**SEQ ID N°15** : amorce sens PCR-B (transcrits IL7R-001)
   CTCTGTCGCTCTGTTGGTC
**SEQ ID N°16** : amorce anti-sens PCR-B (transcrits IL7R-001)
   TCCAGAGTCTTCTTATGATCG
**SEQ ID N°17** : sonde PCR-B (transcrits IL7R-001)
   CTATCGTATGGCCCAGTCTCC
**SEQ ID N°18** : amorce sens PCR-C (transcrits IL7R-007)
   GGAAGTGAATGGATCGCAGC
**SEQ ID N°19** : amorce anti-sens PCR-C (transcrits IL7R-007)
   CAGAATGTCCAGACACAGTG
**SEQ ID N°20** : sonde PCR-C (transcrits IL7R-007)
   CTGTGCTTTTGAGGACCCAGAT

## Revendications

1. Procédé d'évaluation *in vitro* ou *ex vivo* du risque de complication chez un patient ayant subi une agression ou une infection, ladite agression ou infection générant un syndrome de réponse inflammatoire systémique, **caractérisé en ce qu'**il comprend la détection, de préférence la quantification, dans un échantillon biologique issu dudit patient, d'au moins un transcrit du gène *IL7R* correspondant au transcrit IL7R-001 de **SEQ ID N°2** ou à un de ses variants, la séquence d'un variant présentant au moins 99% d'identité avec ladite séquence, et correspondant de préférence au transcrit IL7R-001 de **SEQ ID N°2.**

2. Procédé selon la revendication 1, **caractérisé en ce que** le risque de complication est le risque de décès du patient.

3. Procédé selon la revendication 1 ou 2, **caractérisé en ce que** lorsque la détection, de préférence la quantification, concerne plusieurs transcrits du gène *IL7R,* le(s) transcrit(s), autre(s) que le transcrit IL7R-001 de **SEQ ID N°2** ou qu'un de ses variants, est choisi parmi les transcrits IL7R-002 de **SEQ ID N°3,** IL7R-003 de **SEQ ID N°4,** IL7R-005 de **SEQ ID N°6** et IL7R-007 de **SEQ ID N°8** et leurs variants, la séquence d'un variant présentant au moins 99% d'identité avec l'une desdites séquences.

4. Procédé selon l'une quelconque des revendications 1 à 3, **caractérisé en ce qu'**il met en œuvre les étapes consistant à :
i) déterminer la quantité dudit au moins un transcrit du gène *IL7R* dans ledit échantillon biologique du patient,
ii) comparer la quantité dudit au moins un transcrit déterminée pour ledit échantillon biologique ou une valeur dérivée de cette quantité, à une valeur de référence prédéterminée, et
iii) établir une conclusion quant à la présence éventuelle d'un risque de complication, à partir du résultat de la comparaison.

5. Procédé selon l'une quelconque des revendications 1 à 3, **caractérisé en ce qu'**il met en œuvre les étapes consistant à :
i) déterminer la quantité globale de plusieurs transcrits du gène *IL7R,* dont le transcrit IL7R-001 de **SEQ ID N°2** ou un de ses variants, dans ledit échantillon biologique du patient,
ii) comparer la quantité globale desdits transcrits déterminée pour ledit échantillon biologique ou une valeur dérivée de cette quantité, à une valeur de référence prédéterminée et
iii) établir une conclusion quant à la présence éventuelle d'un risque de complication, à partir du résultat de la comparaison.

6. Procédé selon l'une quelconque des revendications 1 à 5, **caractérisé en ce que** l'échantillon biologique à tester est issu d'un patient présentant un sepsis, notamment un sepsis sévère ou ayant présenté un sepsis, notamment un sepsis sévère, dans les 6 jours précédant le prélèvement de l'échantillon biologique à tester ou d'un patient en état de choc septique ou ayant été en état de choc septique dans les 6 jours précédant le prélèvement de l'échantillon biologique à tester.

7. Procédé selon l'une des revendications 4 à 6, **caractérisé en ce que** la valeur de référence, nommée référence interne, est obtenue à partir d'un échantillon de référence issu du même patient et obtenu lors d'un prélèvement antérieur.

8. Procédé selon la revendication 7, **caractérisé en ce que** le patient a été admis en service de réanimation et l'échantillon de référence interne provient d'un prélèvement antérieur obtenu dans les 2 jours ou dans le jour, ou encore à 2 jours ou à 1 jour après l'admission en réanimation du patientet, le prélèvement de l'échantillon à tester a été effectué à 3 jours après l'admission en réanimation.

9. Procédé selon l'une des revendications 4 à 6, **caractérisé en ce que** la valeur de référence, nommée référence externe, est obtenue à partir d'un échantillon de référence d'un individu de référence ou d'échantillons de référence d'une population de référence.

10. Procédé selon la revendication 9, **caractérisé en ce que** l'échantillon biologique à tester est issu d'un patient en état de choc septique au moment du prélèvement de l'échantillon biologique à tester ou ayant été en état de choc septique dans les 6 jours précédant le prélèvement de l'échantillon biologique à tester et la valeur de référence externe correspond à, ou est dérivée de, la quantité dudit au moins un transcrit du gène *IL7R* mesurée dans un échantillon biologique de référence issu d'un patient en état de choc septique au moment du prélèvement de l'échantillon biologique de référence ou ayant été en état de choc septique dans les 6 jours précédant le prélèvement de l'échantillon biologique à tester, dont on sait qu'il n'a pas connu de complication, et en particulier dont on sait qu'il a survécu, ou bien correspond à une valeur moyenne de la quantité dudit au moins un transcrit du gène *IL7R* qui est mesurée sur un pool d'échantillons de référence issu de patients en état de choc septique au moment du prélèvement desdits échantillons biologiques de référence ou ayant été en état de choc septique dans les 6 jours précédant le prélèvement de desdits échantillons biologiques de référence, dont on sait qu'ils n'ont pas connu de complication, et en particulier dont on sait qu'ils ont survécu.

11. Procédé selon l'une quelconque des revendications 1 à 10, **caractérisé en ce que** la quantité dudit au moins un transcrit du gène *IL7R* est mesurée par RT-PCR quantitative et ce que la quantité dudit au moins un transcrit du gène *IL7R* est mesurée avec au moins l'un des couples d'amorces d'amplification suivants, avec ou sans la sonde d'hybridation mentionnée :
- amorce sens de **SEQ ID N°12** et amorce anti-sens de **SEQ ID N°13,** et éventuellement sonde de **SEQ ID N°14,**
- amorce sens de **SEQ ID N°15** et amorce anti-sens de **SEQ ID N°16,** et éventuellement sonde de **SEQ ID N°17,**
- amorce sens de **SEQ ID N°18** et amorce anti-sens de **SEQ ID N°19,** et éventuellement sonde de **SEQ ID N°20.**

12. Utilisation de la mesure, *in vitro* ou *ex vivo,* dans un échantillon biologique d'un patient ayant subi une agression ou une infection générant un syndrome de réponse inflammatoire systémique, de la quantité d'au moins un transcrit du gène *IL7R* correspondant au transcrit IL7R-001 de **SEQ ID N°2** ou à un de ses variants, la séquence d'un variant présentant au moins 99% d'identité avec ladite séquence, correspondant de préférence au transcrit IL7R-001 de **SEQ ID N°2,** pour évaluer le risque de complication, et en particulier de mortalité, chez ledit patient.

13. Utilisation selon la revendication 12, **caractérisée en ce que** l'échantillon biologique à tester est issu d'un patient en état de choc septique ou ayant été en état de choc septique dans les 6 jours précédant le prélèvement de l'échantillon biologique à tester.

14. Utilisation selon la revendication 12 ou 13, **caractérisée en ce que** la quantité de plusieurs transcrits du gène *IL7R* est mesurée, le(s) transcrit(s), autre(s) que le transcrit IL7R-001 de **SEQ ID N°2** ou un de ses variants, étant choisi parmi les transcrits IL7R-002 de **SEQ ID N°3,** IL7R-003 de **SEQ ID N°4,** IL7R-005 de **SEQ ID N°6** et IL7R-007 de **SEQ ID N°8** et leurs variants.

15. Kit pour la mesure, *in vitro* ou *ex vivo,* de la quantité d'au moins un transcrit du gène *IL7R* dans un échantillon biologique, ledit au moins un transcrit du gène *IL7R* correspondant au transcrit IL7R-001 de **SEQ ID N°2** ou à un de ses variants, la séquence d'un variant présentant au moins 99% d'identité avec ladite séquence, et correspondant de préférence au transcrit IL7R-001 de **SEQ ID N°2,** ledit kit comprenant :
- des outils ou réactifs spécifiques permettant de mesurer la quantité dudit au moins un transcrit du gène *IL7R* dans ledit échantillon biologique, et
- un échantillon contrôle qui est un échantillon calibré pour contenir la quantité dudit au moins un transcrit du gène *IL7R* qui correspond à la quantité moyenne mesurée dans un pool d'échantillons de patients ayant subi une agression ou une infection générant un syndrome de réponse inflammatoire systémique, dont on sait qu'ils n'ont pas connu de complication, et de préférence dont on sait qu'ils ont survécu, et/ou un échantillon contrôle qui est un échantillon calibré pour contenir la quantité dudit au moins un transcrit du gène *IL7R* qui correspond à la quantité moyenne mesurée dans un pool d'échantillons de patients ayant subi une agression ou une infection générant un syndrome de réponse inflammatoire systémique, dont on sait qu'ils ont connu une complication, et de préférence dont on sait qu'ils n'ont pas survécu.

16. Kit selon la revendication 15, **caractérisé en ce qu'**il contient, en tant que réactifs spécifiques permettant de mesurer la quantité dudit au moins un transcrit du gène *IL7R* dans ledit échantillon biologique, au moins l'un des couples d'amorces d'amplification suivants, avec ou sans la sonde mentionnée :
- amorce sens de **SEQ ID N°12** et amorce anti-sens de **SEQ ID N°13,** et éventuellement sonde de **SEQ ID N°14,**
- amorce sens de **SEQ ID N°15** et amorce anti-sens de **SEQ ID N°16,** et éventuellement sonde de **SEQ ID N°17,**
- amorce sens de **SEQ ID N°18** et amorce anti-sens de **SEQ ID N°19,** et éventuellement sonde de **SEQ ID N°20.**

## Patentansprüche

1. *In-vitro-* oder *Ex-vivo*-Verfahren zum Bewerten des Komplikationsrisikos bei einem Patienten, der einen Angriff oder eine Infektion erlitten hat, wobei der Angriff oder die Infektion ein systemisches inflammatorisches Response-Syndrom erzeugt, **dadurch gekennzeichnet, dass** es das Erfassen, vorzugsweise das Quantifizieren, in einer biologischen Probe, die von einem Patienten stammt, mindestens eines Transkripts des *IL7R-*Gens aufweist, das dem Transkript IL7R-001 von SEQ ID Nr. 2 oder einer seiner Varianten entspricht, wobei die Sequenz einer Variante mindestens 99 % Identität mit der Sequenz aufweist und vorzugsweise dem Transkript IL7R-001 von SEQ ID Nr. 2 entspricht.

2. Verfahren nach Anspruch 1, **dadurch gekennzeichnet, dass** das Komplikationsrisiko das Risiko des Todes des Patienten ist.

3. Verfahren nach Anspruch 1 oder 2, **dadurch gekennzeichnet, dass**, wenn das Erfassen, vorzugsweise das Quantifizieren, mehrere Transkripte des *IL7R-*Gens betrifft, das (die) andere(n) Transkript(e) als das Transkript IL7R-001 von SEQ ID Nr. 2 oder als eine seiner Varianten aus den Transkripten IL7R-002 von SEQ ID Nr. 3, IL7R-003 von SEQ ID Nr. 4, IL7R-005 von SEQ ID Nr. 6 und IL7R-007 von SEQ ID Nr. 8 und ihren Varianten ausgewählt wird (werden), wobei die Sequenz einer Variante mindestens 99 % Identität mit einer der Sequenzen aufweist.

4. Verfahren nach einem der Ansprüche 1 bis 3, **dadurch gekennzeichnet, dass** es die Schritte umsetzt, die darin bestehen:
i) die Menge des mindestens einen Transkripts des *IL7R-*Gens in der biologischen Probe des Patienten zu bestimmen,
i) die Menge des mindestens einen Transkripts, das für die biologische Probe des Patienten bestimmt wird, oder einen Wert, der von dieser Menge abgeleitet wird, mit einem vorbestimmten Referenzwert zu vergleichen,
iii) eine Schlussfolgerung hinsichtlich der eventuellen Anwesenheit eines Komplikationsrisikos ausgehend von dem Ergebnis des Vergleichs zu ziehen.

5. Verfahren nach einem der Ansprüche 1 bis 3, **dadurch gekennzeichnet, dass** es die Schritte umsetzt, die darin bestehen:
i) die Gesamtmenge von mehreren Transkripten des IL7R-Gens, darunter das Transkript IL7R-001 von SEQ ID Nr. 2 oder einer seiner Varianten in der biologischen Probe des Patienten zu bestimmen,
ii) die Gesamtmenge der Transkripte, die für die biologische Probe des Patienten bestimmt wird, oder einen Wert, der von dieser Menge abgeleitet wird, mit einem vorbestimmten Referenzwert zu vergleichen und
iii) eine Schlussfolgerung hinsichtlich der eventuellen Anwesenheit eines Komplikationsrisikos ausgehend von dem Ergebnis des Vergleichs zu ziehen.

6. Verfahren nach einem der Ansprüche 1 bis 5, **dadurch gekennzeichnet, dass** die zu testende biologische Probe von einem Patienten, der eine Sepsis, insbesondere eine schwere Sepsis, aufweist, oder eine Sepsis, insbesondere eine schwere Sepsis, innerhalb von 6 Tagen vor der Entnahme der zu testenden biologischen Probe aufgewiesen hat, oder von einem Patienten stammt, der sich im septischen Schock befindet oder der sich innerhalb von 6 Tagen vor der Entnahme der zu testenden biologischen Probe im septischen Schock befand.

7. Verfahren nach einem der Ansprüche 4 bis 6, **dadurch gekennzeichnet, dass** der Referenzwert, der interne Referenz genannt wird, aus einer Referenzprobe erhalten wird, die von dem gleichen Patienten stammt und bei einer vorherigen Entnahme erhalten wird.

8. Verfahren nach Anspruch 7, **dadurch gekennzeichnet, dass** der Patient auf die Intensivstation aufgenommen wurde und die interne Referenzprobe von einer vorherigen Entnahme stammt, die innerhalb von 2 Tagen oder innerhalb eines Tages oder auch 2 Tage oder 1 Tag nach der Aufnahme auf die Intensivstation des Patienten erhalten wurde, die Entnahme der zu testenden biologischen Probe 3 Tage nach der Aufnahme auf die Intensivstation durchgeführt worden ist.

9. Verfahren nach einem der Ansprüche 4 bis 6, **dadurch gekennzeichnet, dass** der Referenzwert, der externe Referenz genannt wird, von einer Referenzprobe einer Referenzperson oder von Referenzproben einer Referenzpopulation erhalten wird.

10. Verfahren nach Anspruch 9, **dadurch gekennzeichnet, dass** die zu testende biologische Probe von einem Patienten, der sich zum Zeitpunkt die Entnahme der zu testenden biologischen Probe im septischen Schock befindet oder der sich innerhalb von 6 Tagen vor der Entnahme der zu testenden biologischen Probe im septischen Schock befand, stammt und der externe Referenzwert der Menge des mindestens einen Transkripts des *IL7R-*Gens entspricht oder davon abgeleitet wird, die in einer biologischen Referenzprobe gemessen wird, die von einem Patienten stammt, der sich zum Zeitpunkt die Entnahme der biologischen Referenzprobe im septischen Schock befindet oder der sich innerhalb von 6 Tagen vor der Entnahme der zu testenden biologischen Probe im septischen Schock befand, von dem bekannt ist, dass sie keine Komplikation erlitten haben, und von dem insbesondere bekannt ist, dass er überlebt hat, oder auch einem Durchschnittswert der Menge des mindestens einen Transkripts des *IL7R-*Gens entspricht, der an einem Pool von Referenzproben gemessen wird, der von Patienten stammt, die sich zum Zeitpunkt die Entnahme der biologischen Referenzproben im septischen Schock befinden oder die sich innerhalb von 6 Tagen vor der Entnahme der zu testenden biologischen Referenzproben im septischen Schock befanden, von denen bekannt ist, dass sie keine Komplikation erlitten haben, und von denen insbesondere bekannt ist, dass sie überlebt haben.

11. Verfahren nach einem der Ansprüche 1 bis 10, **dadurch gekennzeichnet, dass** die Menge des mindestens einen Transkripts des *IL7R-*Gens durch quantitative Echtzeit-PCR gemessen wird und dass die Menge des mindestens einen Transkripts des *IL7R-*Gens mit mindestens einem der folgenden Amplifikationsprimerpaaren mit oder ohne die genannte Hybridisierungssonde gemessen wird:
- Vorwärtsprimer von SEQ ID Nr. 12 und Rückwärtsprimer von SEQ ID Nr. 13 und gegebenenfalls Sonde von SEQ ID Nr. 14,
- Vorwärtsprimer von SEQ ID Nr. 15 und Rückwärtsprimer von SEQ ID Nr. 16 und gegebenenfalls Sonde von SEQ ID Nr. 17,
- Vorwärtsprimer von SEQ ID Nr. 18 und Rückwärtsprimer von SEQ ID Nr. 19 und gegebenenfalls Sonde von SEQ ID Nr. 20.

12. Verwendung der *In-vitro-* oder *Ex-vivo*-Messung in einer biologischen Probe von einem Patienten, der einen Angriff oder eine Infektion erlitten hat, die ein systemisches inflammatorisches Response-Syndrom erzeugt, der Menge mindestens eines Transkripts des *IL7R-*Gens, das dem Transkript IL7R-001 von SEQ ID Nr. 2 oder einer seiner Varianten entspricht, wobei die Sequenz einer Variante mindestens 99 % Identität mit der Sequenz aufweist, die vorzugsweise dem Transkript IL7R-001 von SEQ ID Nr. 2 entspricht, um das Komplikationsrisikos und insbesondere das Mortalitätsrisiko bei dem Patienten zu bewerten.

13. Verwendung nach Anspruch 12, **dadurch gekennzeichnet, dass** die zu testende biologische Probe von einem Patienten stammt, der eine Sepsis aufweist oder eine Sepsis innerhalb von 6 Tagen vor der Entnahme der zu testenden biologischen Probe aufgewiesen hat.

14. Verwendung nach Anspruch 12 oder 13, **dadurch gekennzeichnet, dass** die Menge von mehreren Transkripten des *IL7R-*Gens gemessen wird, wobei das (die) andere(n) Transkript(e) als das Transkript IL7R-001 von SEQ ID Nr. 2 oder als eine seiner Varianten aus den Transkripten IL7R-002 von SEQ ID Nr. 3, IL7R-003 von SEQ ID Nr. 4, IL7R-005 von SEQ ID Nr. 6 und IL7R-007 von SEQ ID Nr. 8 und ihren Varianten ausgewählt wird (werden).

15. Kit zur *In-vitro-* oder *Ex-vivo*-Messung der Menge mindestens eines Transkripts des *IL7R*-Gens, das dem Transkript IL7R-001 von SEQ ID Nr. 2 oder einer seiner Varianten entspricht, in einer biologischen Probe, wobei das mindestens eine Transkript des *IL7R-*Gens dem Transkript IL7R-001 von SEQ ID Nr. 2 oder einer seiner Varianten entspricht, wobei die Sequenz einer Variante mindestens 99 % Identität mit der Sequenz aufweist, und vorzugsweise dem Transkript IL7R-001 von SEQ ID Nr. 2 entspricht, wobei der Kit aufweist:
- spezifische Werkzeuge oder Reagenzien, die ermöglichen, die Menge des mindestens einen Transkripts des *IL7R-*Gens in der biologischen Probe zu messen, und
- eine Kontrollprobe, die eine Probe ist, die kalibriert ist, um die Menge des mindestens einen Transkripts des IL7R*-*Gens zu enthalten, die der Durchschnittsmenge entspricht, die in einem Pool von Proben von Patienten gemessen wird, die einen Angriff oder eine Infektion erlitten haben, die ein systemisches inflammatorisches Response-Syndrom erzeugt, von denen bekannt ist, dass sie keine Komplikation erlitten haben, und von denen vorzugsweise bekannt ist, dass sie überlebt haben, und/oder eine Kontrollprobe, die eine Probe ist, die kalibriert ist, um die Menge des mindestens einen Transkripts des *IL7R-*Gens zu enthalten, die der Durchschnittsmenge entspricht, die in einem Pool von Proben von Patienten gemessen wird, die einen Angriff oder eine Infektion erlitten haben, der oder die ein systemisches inflammatorisches Response-Syndrom erzeugt, von denen bekannt ist, dass sie eine Komplikation erlitten haben, und von denen vorzugsweise bekannt ist, dass sie nicht überlebt haben.

16. Kit nach Anspruch 15, **dadurch gekennzeichnet, dass** es als spezifische Reagenzien, die ermöglichen, die Menge des mindestens einen Transkripts des IL7R-Gens in der biologischen Probe zu messen, mindestens eines der folgenden Amplifikationsprimerpaare mit oder ohne die genannte Hybridisierungssonde enthält:
- Vorwärtsprimer von SEQ ID Nr. 12 und Rückwärtsprimer von SEQ ID Nr. 13 und gegebenenfalls Sonde von SEQ ID Nr. 14,
- Vorwärtsprimer von SEQ ID Nr. 15 und Rückwärtsprimer von SEQ ID Nr. 16 und gegebenenfalls Sonde von SEQ ID Nr. 17,
- Vorwärtsprimer von SEQ ID Nr. 18 und Rückwärtsprimer von SEQ ID Nr. 19 und gegebenenfalls Sonde von SEQ ID Nr. 20.

## Claims

1. A method of *in vitro* or *ex vivo* evaluation of the risk of complications in a patient who has sustained an insult or an infection, said insult or infection generating a systemic inflammatory response syndrome, the method being **characterized in that** it comprises detecting, preferably quantifying, in a biological sample obtained from said patient, at least one transcript of the *IL7R* gene corresponding to the transcript IL7R-001 of **SEQ ID NO: 2** or to one of its variants, the sequence of a variant having at least 99% identity with said sequence, and preferably corresponding to the transcript IL7R-001 of **SEQ ID NO: 2.**

2. A method according to claim 1, **characterized in that** the risk of complications is the risk of death of the patient.

3. A method according to claim 1, 2, or 3, **characterized in that**, when the detection, preferably the quantification, concerns several transcripts of the *IL7R* gene, the said transcript(s) different from the transcript IL7R-001 of **SEQ ID NO:** 2 or one of its variants, is selected from the transcripts IL7R-002 of **SEQ ID NO: 3,** IL7R-003 of **SEQ ID NO: 4,** IL7R-005 of **SEQ ID NO: 6,** and IL7R-007 of **SEQ ID NO: 8** and their variants, the sequence of a variant having at least 99% identity with one of said sequences.

4. A method according to any one of claims 1 to 3, **characterized in that** it employs the steps consisting in:
i) determining the quantity of said at least one transcript of the *IL7R* gene in said biological sample from a patient;
ii) comparing the quantity of said at least one transcript determined for said biological sample or a value derived from this quantity with a predetermined reference value; and
iii) from the result of the comparison, coming to a conclusion as to the possible presence of a risk of complications.

5. A method according to any one of claims 1 to 3, **characterized in that** it implements the steps consisting in:
i) determining the overall quantity of a plurality of transcripts of the *IL7R* gene, including the transcript IL7R-001 of **SEQ ID NO: 2** or one of its variants, in said biological sample from a patient;
ii) comparing the overall quantity of said transcripts determined for said biological sample, or a value derived from this quantity, with a predetermined reference value; and
iii) from the result of the comparison, coming to a conclusion as to the possible presence of a risk of complications.

6. A method according to any one of claims 1 to 5, **characterized in that** the biological test sample is obtained from a patient presenting a sepsis, in particular a severe sepsis, or who has previously presented a sepsis, in particular a severe sepsis, within the 6 days preceding taking the biological test sample or from a patient in a state of septic shock or who has been in a state of septic shock within the 6 days preceding taking the biological test sample.

7. A method according to any one of claims 4 to 6, **characterized in that** the reference value, termed the internal reference, is obtained from a reference sample obtained from the same patient and obtained from an earlier taking.

8. A method according to claim 7, **characterized in that** the patient has been admitted to an intensive care unit and the internal reference sample originates from an earlier taking obtained within the 2 days or within a day, or indeed on day 2 or on day 1 following admission of the patient to intensive care and the taking of the test sample is carried out on day 3 following admission to intensive care.

9. A method according to any one of claims 4 to 6, **characterized in that** the reference value termed the external reference is obtained from a reference sample for a reference individual or from reference samples for a reference population.

10. A method according to claim 9, **characterized in that** the biological test sample is obtained from a patient in a state of septic shock at the time the biological test sample is taken or who has been in a state of septic shock within the 6 days preceding taking the biological test sample, and the external reference value corresponds to or is derived from the measured quantity of said at least one transcript of the *IL7R* gene measured in a biological reference sample obtained from a patient in a state of septic shock at the time the biological reference sample is taken or who has been in a state of septic shock within the 6 days preceding taking the biological test sample, who is known not to have suffered any complications, and in particular who is known to have survived, or indeed corresponds to a mean value of the quantity of said at least one transcript of the *IL7R* gene that is measured in a pool of reference samples obtained from patients in a state of septic shock at the time said reference biological samples were taken or who have been in a state of septic shock within the 6 days preceding taking said reference biological samples, who are known not to have suffered any complications, and in particular who are known to have survived.

11. A method according to any one of claims 1 to 10, **characterized in that** the quantity of said at least one transcript of the IL7R gene is measured by quantitative RT-PCR and **in that** the quantity of said at least one transcript of the IL7R gene is measured with at least one of the following amplification primer pairs, with or without the hybridization probe mentioned:
- forward primer with **SEQ ID NO: 12** and reverse primer with **SEQ ID NO: 13,** and optionally probe with **SEQ ID NO: 14;**
- forward primer with **SEQ ID NO: 15** and reverse primer with **SEQ ID NO: 16,** and optionally probe with **SEQ ID NO: 17;**
- forward primer with **SEQ ID NO: 18** and reverse primer with **SEQ ID NO: 19,** and optionally probe with **SEQ ID NO: 20.**

12. Use of the *in vitro* or *ex vivo* measurement, in a biological sample from a patient who has sustained an insult or an infection generating a systemic inflammatory response syndrome, of the quantity of at least one transcript of the *IL7R* gene corresponding to the transcript IL7R-001 of **SEQ ID NO: 2** or to one of its variants, the sequence of a variant having at least 99% identity with said sequence, and preferably corresponding to the transcript IL7R-001 of **SEQ ID NO: 2,** in order to evaluate the risk of complications, and in particular of mortality, in said patient.

13. Use according to claim 12, **characterized in that** the biological test sample is obtained from a patient in a state of septic shock or who has been in a state of septic shock within the 6 days preceding taking the biological test sample.

14. Use according to claim 12 or 13, **characterized in that** the quantity of several transcripts of the *IL7R* gene is measured, the transcript(s) different from the transcript IL7R-001 of **SEQ ID NO: 2** or one of its variants being selected from IL7R-002 of **SEQ ID NO: 3,** IL7R-003 of **SEQ ID NO: 4,** IL7R-005 of **SEQ ID NO: 6,** and IL7R-007 of **SEQ ID NO: 8** and their variants.

15. A kit for the *in vitro* or *ex vivo* measurement of the quantity of at least one transcript of the IL7R gene in a biological sample, the said at least one transcript of the *IL7R* gene corresponding to the transcript IL7R-001 of **SEQ ID NO: 2** or to one of its variants, the sequence of a variant having at least 99% identity with said sequence, the said kit comprising:
- specific reagents or tools for measuring the quantity of said at least one transcript of the *IL7R* gene in said biological sample; and
- a control sample that is a sample that is calibrated in order to contain the quantity of said at least one transcript of the *IL7R* gene that corresponds to the mean quantity measured in a pool of samples from patients who have sustained an insult or an infection generating a systemic inflammatory response syndrome, who are known not to have suffered any complications, and preferably who are known to have survived, and/or a control sample that is a sample that is calibrated in order to contain the quantity of said at least one transcript of the *IL7R* gene that corresponds to the mean quantity measured in a pool of samples from patients who have sustained an insult or an infection generating a systemic inflammatory response syndrome, who are known to have suffered complications, and preferably who are known not to have survived.

16. A kit according to claim 15, **characterized in that** it contains, as specific reagents for measuring the quantity of said at least one transcript of the IL7R gene in said biological sample, at least one of the following pairs of amplification primers, with or without the probe mentioned:
- forward primer with **SEQ ID NO: 12** and reverse primer with **SEQ ID NO: 13,** and optionally probe with **SEQ ID NO: 14;**
- forward primer with **SEQ ID NO: 15** and reverse primer with **SEQ ID NO: 16,** and optionally probe with **SEQ ID NO: 17;**
- forward primer with **SEQ ID NO: 18** and reverse primer with **SEQ ID NO: 19,** and optionally probe with **SEQ ID NO: 20.**


### SEQUENCE LISTING

<110> bioMérieux HOSPICES CIVILS DE LYON
<120> Procédé dévaluation du risque de complications chez les patients qui présentent un syndrome de réponse inflammatoire systémique (SIRS)
<130> 1H146190-BFR-0066
<160> 20
<170> BiSSAP 1.3
<210> 1
   <211> 22729
   <212> DNA
   <213> Artificial Sequence
<220>
   <223> IL7R gene
<400> 1
   atctaagctt ctctgtcttc ctccctccct cccttcctct tactctcatt catttcatac      60
   acactggctc acacatctac tctctctctc tatctctctc agaatgacaa ttctaggtac     120
   aacttttggc atggtttttt ctttacttca agtcgtttct ggagaaagtg gctatgctca     180
   aaatggtgag tcatttctaa gttttcttat ggattttgga ttatctgtag catggtttca     240
   ggttattcag ttccctaaca gacctgagtc aggcactggg tttgaatgca gtttgagaat     300
   ttcccacata ttcagtcatt ttttttaatg tttaaccacc atgacagggg gcaggggatc     360
   aatactatgg gtggtttata agacctcagt attctcaaga aggaatgcat ttcactccca     420
   agtgtagatc ttaaatgttg aatgattact ctgctcttac aaaaagaatg ctcatgtaga     480
   tgctatgact gtacttgtag gaaaatgtcc aaagtaattt taccttgtca ggagatcaaa     540
   ctggattcat tttgtttgac tttttaagaa atcctgaaag cataactttc aggataaggt     600
   aatgtacaga agcaatagct ttgtcttcag tgaccagtgc tatatcctca gcacctaaat     660
   cagtggctag aatatagtag acatccaata acttttgaaa gtgttttcaa aatactttag     720
   ttttgagaga tttatgtgag attttaagta aataactgac tagagaaaga tctaaatgag     780
   tttactcatt gaaatacact gaattgcctc cacaccaaca aattggccat atgtaataat     840
   tctttttggg atctaaaaaa cttagtaccg agaagccaac cctgcccata cataaacaca     900
   ttgtaattat aacaaaacta ggcagaagct tctaacagca gcaggaggca tgtgggaatt     960
   tagaccatca acttgctcct gcaaattaag ccctttctct ttaagagtta aaaactattt    1020
   ggctatagac aatatcaaac acatcagcct aatgactcag cttatgcatt ttgagtcatg    1080
   taattacgaa ggatggaaat ccctagaatt ttctcattaa gggaattgtc agagagtttg    1140
   acatttttta cagtatatga ctcactttat gggggatgat tattattcta tgctaaactt    1200
   tgccttggat ttccacaaag actgatggga ggcaggaaac ataaatctta ctctctttca 1260
   tgtcatctat actcactagt tcaccctggt gatcatacta tttttaaaat atataagaat 1320
   gctagttgaa agctgggttt tcactccaac tttttaagtt tcagattttt tagaagatgt 1380
   ataattaccc tattcacatg attacgtcaa aatacttccc agtttggggt ataggaattc 1440
   acattcagtt gctgcttgtt gaaagttgtc aattttctga tcatcacaag gatgatcaag 1500
   agaagaaagg gatacttttt aaaaatccaa atcatttaca ctattaatca actaactcca 1560
   ttcagtagga agaagacttc tagatgacac tggcttgcct atgatacata ttccacacaa 1620
   tttaaatttt tatggataaa tatgtctaga tacctattta aatatgaata atattaatta 1680
   ttgagcattt aaagaataat agattaactc attattcaaa agctctatgt aatttcaaaa 1740
   ccatagtaat tataacaccg tcaattgaca taaacttttt aaagagaagc tcaaatgttt 1800
   catgtatatt ttcagaatta gaattcttat tttacctttt cattacttat ttctcagaaa 1860
   atattatact catagctaat ccctattaaa tccttactgt gttctaagct acctctttgt 1920
   aaatatccat tcagtgattg ctcatagcac gagtttacat attagaacac atgtcttaga 1980
   gaagttgcct acctgacaga ggaccacagg tagagtatcc agaatttaaa cgcacatctg 2040
   tccagctcta acaccacagg tcttaaccac tgtgtacatt aactactctt agccaagaat 2100
   ttttcagctc acgtcatgta gaatattctt tttgtaaaat gccatcacat tttataagtc 2160
   attgaaggga atttttcttg gttacaaagc aactctgccc cataatatct actgaaaagc 2220
   cagtgagctg cttcctaaaa cacagccatt ttaggtgcag gaaacagtgt ataaatggct 2280
   cattgtatat tgtatgcttt gccagactga gtggcagtgg gagtcctttg ttatgtgggt 2340
   gctgacatct gctagagtgt gctgtctcta ttgaagaatc gtgaagacaa agccgaccca 2400
   caggatgtct gaatccaaat aataatacat gttctgtgta tagaattggt ggaagagaaa 2460
   atgtcaggac agtgtgagga ctgccatgta aggtcagaac cactgcattt agaaagctac 2520
   cactgcacag ggaagaaatc taagtctaca aaattagtgg gctgtctctc attatttcgt 2580
   gctgtcatca gaaggagggc cataccctgc tgaaactaca taaagagctt ttgctggtgg 2640
   cagaactgtg aactggatgg attctgggaa tggccagaaa aacaaatgcc tgtggttgtg 2700
   agcagtgccc acacccatgg tctagctagg gctgtttgag atttgttgct ttgactgaac 2760
   caacctgtca ttcaactggt tggtccattc acagtcagct ttattaactt tcccattttc 2820
   cctactgagt tatttaagta aagaaagtgc tattcggaca gcccttggtc tctgggacaa 2880
   tcaactggga tttgatttta gtatattctg tctccagtgt aaagccttgg aagcatctaa 2940
   tttctagtac tgatgaacca aaaatacatg gaagcagtcc taggctcaca cttgagcact 3000
   ctgagaatgg ctttgcttac tccagatttt ctcaggtccc agtgggtgta tattttctga 3060
   catatttatt ccagcctcac tttctatcat gtaaaacata catacaaaat gtagatttca 3120
   ttatagggtc tacaaaacag cttaagaaac caaatactat gtgtgacaga tcacactttc 3180
   caaaagtaat agcaaaaaaa aaaaaaatct ggttccccac tttcttccag catcctgcta 3240
   gaatctatca gatactgcgt ctatagaaga atctataaga acagaagcag tatgtacaac 3300
   attcacagga agtttcacca aatcggagtc ctgccagatc taattttttt tccctaatca 3360
   cgtttgtctc agtcagtagc ttaagacaat ggaaataatc agtgccactt ttaattggga 3420
   tgccttttta ggcaagggaa agtgacctct taaaaaagca aaattctgac tgcaagatag 3480
   ctatcattgt ccttcattta agacaaaaaa aatactaggg agggaataaa ttatgatttg 3540
   taataaagtg aaaagtgaga ttaggtagca tggggataat ggaaataaag tgtctcttct 3600
   ttgaaataat atgaacaatc aatgtaacaa atgtagcaga aaaaactcca gtttaaatac 3660
   agaaaagaat gtgttcaatg cctctggttc tttaactcag aaatatttgg aggttactta 3720
   ctcattatga tggatttttt ttttctattg gaaaactctg ttagcattga gcgtttttgt 3780
   tttttgtttt ttgttggttg gttggttttg aagcattttt cttgtctttg cccttgggct 3840
   tttcttcctt gaatactaca taatccatta ctatttcatg tctgccacag agtctgctat 3900
   tttattaagg tcatgccata tttcaaaagg atgcatttat ttgtttcatt aacagctgca 3960
   tgtttgttcc tccccaggag acttggaaga tgcagaactg gatgactact cattctcatg 4020
   ctatagccag ttggaagtga atggatcgca gcactcactg acctgtgctt ttgaggaccc 4080
   agatgtcaac atcaccaatc tggaatttga aatatggtga gggatggtgg ttttaatggt 4140
   tgcttagaca tcctctgtct ctcttttcat atgctctttt taatagccac aaaagaaaga 4200
   atatgtggcc taattaacaa atgttaacat ctaaggaatt cccaaaggcc tcctgaaact 4260
   ccttgtcctt caccaaaaac actcatacaa atctcctctc acggttcagc tttcagaccc 4320
   tgagactcag tcaaatgatg ctctggatct tggggatccc acatccctcc caacttcata 4380
   tcagaattta aatcctgcgt ctcctacaac acttctcacc aaaaatctgt ttgcccaaca 4440
   cgagacaatc cagtgtcttc aagttgcatc tgagagttaa actgccttgt ttccaatccc 4500
   aataccagtg cttactagtt ttttgaccta gagaaagtta tgtaatgtat ctatgcctca 4560
   gtttcctcac ctgtaaaatg agataacctg cctcacagga aggctgtgat ggttaaataa 4620
   tttcatcata taaatcattc caaatagtcg gccagtgaat aacgagtaat ggggaagcaa 4680
   cattaaatta taattctgtg aatattgacc taacttctac catcttgaca caatttgact 4740
   tcagatgatc ctctcaatgt aaattttcca aaaatccaca ggaataagtt ggcattttgt 4800
   ttcacaaggt ctcacagaaa agacaaagga aaagagtctg gtttgaaagt ttactaaagg 4860
   tctcagggaa ctttatcttc tccttctcct tcatccataa gtcatctctt gttgccaagg 4920
   gttactatct ctggtgattt gagaaactac tctagcttga aattctgacc tgaggctatc 4980
   tccaaattca tatccgaatg acctactttt tagttagtgt cctagtgagc aaagtaaatc     5040
   aagatccacc agtagtaata gaaggcttcc tacattccat agacactgag acaattctcc     5100
   acagtctata gtccaaacaa gccctgaatt ccagtttttg tcaatttatg ggagcttcct     5160
   gcatctattt atggagtgct ttctgctgca gtccttagat aaacatgctg ttggacttga     5220
   gtagtgtact gtgttctctg tctgcctctg ttcacttccc taacacattt tccaggaata     5280
   aaatatgtca aaagaacctg aaccagttcg atgtccacaa tctaggctgg aaatggattg     5340
   cactaaaaca gccataacaa ctcattcaaa caaggcactc attttcatgg gcaaatcact     5400
   ctcccacacg gaggtttgac tttggcttct ttaaccagct ggctggtggg ctgagtgttc     5460
   atcctggttt ctcttggcca agctgaggtt gacctttctg ttcactttca ttcacaccat     5520
   atttgaccac ttccttgccc actcaaacat acttaccctt taacatatct cttgactttt     5580
   cctgtcatat tgtaatctgt ccagagcctc ctctatttgg gttttccaat tggattcaga     5640
   tatttcagtt ggaaagggac tgccttaaga aagaaacgtt ttcagtggaa aatatatgta     5700
   tgagctcttt aatagatgaa ctcctggagt tcagagccct taaaaggatg cccagtttca     5760
   caagacagcc atacggtcat ccttgattgt ccattgctca ttaatttcat tctcaaaatc     5820
   atgggaatga gctgagaata ccattttaga tcctccttaa attcccaaca gtaccagaaa     5880
   cttgctacag gttggggcct gtaattggat atttcacaca tactttcctt acaaatatat     5940
   tctatactca agaattgaac taaaagttat tgtcctagtt tctccacatc ccatgtttac     6000
   ctaaaattca gaaatgggac cccgctccca gtctcccctt ctatatttat ttatcaaatc     6060
   gtgacaacat taccatcttc agatctttcc acctgatgtt tgtcctaagc ttattccctg     6120
   gtatctgtct agcttaccca aaaattcggt ttttattttt atcctgttcc aagttgggaa     6180
   agcctatcta ccccaacaag gaacacaact ccctagtaac tttgagacac acacacacat     6240
   acacacctac tctttaaagc ctaaacaatc gcacactcta aaagatagca gttaacaaaa     6300
   gtaacgattt gggagaacag ttttaaggaa tgtccccaaa ataatcaata catttagcca     6360
   gttaattaac ttaacatttc ttcaccaatc tctagttttc atgactgtag gagcttaacc     6420
   agtcactctc agaccacaat aaaccaaagg tgaaagattc tgtaacaaaa gctagggcac     6480
   tctcccctgc atttaacctc ctggccagct cactcgaagc cagacaaaca ggttcctctt     6540
   tttgtgcaga gtccaggaac cattctcgaa aggactcatt tgagcacatg cagagaagag     6600
   tgtacacaca tccagttcac caagggaagc caacacacat tgtgggttgt aggtagtaaa     6660
   aggccttcct agaacacact ccttaggatt taaacaaaat tacatcggtt aatggaaaga     6720
   attctttcat atacgcaaac ttacccagag gaacttttct tctgcccaga tcttcacttc     6780
   caatttgacc cagttatacc tctttagagc tatttggctg agcttaaaca gcacatagga     6840
   aaaacaaatt ggtaactgtg tttatcacag aagaggaaaa ttaaatttag ggttgggaaa     6900
   ggaaaataac cctatgatat tacttttatt ctacctttac aatgagaata tatacctttg 6960
   ttacttcttt aatttttaca ttatttactt atttttcttt gctttcttgt ttgattacaa 7020
   tgcattttag gggtaaaatt tatgtgtggt aaaatgcaca aaaattaagt gaatttggag 7080
   aaatgtctat gacctgtagc cattccaatg gtaaagatat agaacttatt tttcccctag 7140
   aaggatgctt catgttcctt tccagtcaat cttcataccc caggagcaat cataattctc 7200
   aattctatta ccctttggtt tttgccagtt tctgatagtt cttattaata gaatactctt 7260
   tattcttttc tgtcttcttt catttaacca gtgtttgtga gagttagcca tgttgatgtc 7320
   catctcatag ctcatctttt caattgctaa gtagtaattc cactgtatga atataccaca 7380
   aatttttaat tctttctctt cttgatgaac atttgtgttt tttcaagttt gagactatta 7440
   ttttttaggt tgctgttcac attcttggac aaatcagttt gtgtatatat attttcattt 7500
   ttctggggta taaaacctca gaatggaatt gctgtgtcat aaggtaagca tgtatctaag 7560
   tttataagaa accgcccaac agtttttcaa agtggttata ccattctact ctccttccag 7620
   cgatgcatga gagatataca tcatttgcaa cgtttgactt tgggatagta tctcgttagg 7680
   tttttaattc gcatttgtca aataacaaat gttgagcagc ttttcatata cttggtcttt 7740
   tgcctgtctt ctttgggcta gtatctgtta aaagcactga gttatttgtc cttttgttat 7800
   tgctggatat gagttcttta tacattctgt atacatttcc tttgtcagat agatgtattg 7860
   catctatttt ctattctgaa gtttgccatt ttattttctt actggtgcgt tttaataagc 7920
   aagagttttt ttttattttg atggagtcta atatatcatt tattttcttt tatatgtagt 7980
   gctttttgta tccttgctaa gataactttg cctactccca aagttgggaa gatattttct 8040
   catgttttct tttaaatgtt ctacagtttt agcctttata tttagttttt ttaattatta 8100
   ttatacttta agttctaggg tacatgtgca caacgtgtag gtttgttaca tatgtataca 8160
   tgtgccatgt tggtgtgctg caccgattaa ctcgtcattt acattaggta tatctcctaa 8220
   tgctatccct cccccctcct tccacctatg actggccctg gtgtgtgatg ttccccttcc 8280
   tgtgtccaag tgctcttatc gttcaattcc catctatgag tgagaacatg cagtgtttga 8340
   ttttttgtcc ttgtgatagt ttgctgagaa tgatggtttc cagcttcatc catgtcccta 8400
   taaaggacat gaactcatcc ttttttatgg ctgcatagta ttccatggtg tatatgtgcc 8460
   acattttctt aatccagtct atcattgatg gacatttggc ttggttccaa gtctttgcta 8520
   ttgtgaatag tgctgcaata atcgtacatg tgcatgtgtc tttatagcag catgatttat 8580
   actcctttgg gtatataccc agtaatggga tggctgggtc aaatagtatt tctagctctg 8640
   gatccttgag gactcgccac actgtcttcc acaatggttg aactagttta cagtcccacc 8700
   aacagtgtaa aagtgttcct atttctccac attccctcca gcaccttttg tttcctgact 8760
   ttttaatgat caccattcta actggtgtga gatggtatgt cattgtggtt ttgatttgca 8820
   tttctctgat ggccattgat ggctaatatc cagaatctac aatgaactca aacaaattta 8880
   caagaaaaaa acaaacaacc ccatcaaaaa gtgggcaaag gatatgaaca gacacttctc 8940
   aaaagaagac atttatgcag ccaaaagaca catgaaaaaa tgctcatcat caatggccat 9000
   cagagaaacg caaatcaaat tgtgtttatt tgtttctctt gtcttatgca ttggctaaaa 9060
   cctcctgtac accactgaat agaaatggtg aaagtggata ttcctgtcgt gtcctggtct 9120
   tagggaaaca attcatgttc acaatttcag cactaaatat gatattaact ataggctttt 9180
   gtaaatgctc tttatcagat tgaggaagtg tctttctatt tcttatttgc tgtgagtttt 9240
   taacatgaat agatgcattc atgttattaa attatgcttt gaatgcattg attgattata 9300
   accaggttat ttatgtcttc tagtctgtta acatggcaaa ttatattgat taatttttga 9360
   atctttaacc tgctttggtt tcctgagatg tgccctactt tataattatg tattaaaatt 9420
   agtgtgttag tattttcttg tgaaagtttg cttatacatt tttgagggat atttgtctat 9480
   caacttcttt tctctaatat tttggccagg tttgggtacc aggattaagc tagcttcaaa 9540
   aaataggttg agaagggtca ttcctcttcc agtttctaaa ataatttgtg tcagattgac 9600
   actatttctt tccttataca tttgatagaa tttaccagaa tataaccatc aagcatagag 9660
   ttttctttgg ggggaagttt attgataata agtttaattt ctttgagaga aatataactg 9720
   ttgaaatatt ccatttctat gtgggtcaga tttactaatt tgtgtttata aaaacatttt 9780
   cattacatct aagttattat atacattaaa atagcattta aaatttcctt attatacttt 9840
   taacatctgc atgttctata gtgatatctc ctcttacatt ccagatatta gtaatttata 9900
   tattttgttt tcttaaccac tcttgttagg gttcaccagc caaaattacc tataaaaatc 9960
   cattacgtta cccatcaagt atatgtgata ttatgtatat aaccctttat actatgttat 10020
   cattttcttt aacacttttt ttaatcaata ttttttacag ctcttatttc ttacatatat 10080
   tcctatggaa catcaaaaaa agcaattact ttttaatcta aacaaagtat ttgtttttca 10140
   gtgatcaatt ataaaaatat agaaatttcc cataatttta taaatatgtc ttgactattt 10200
   caggttcaat tgcatctaat tctaagtaaa tcatcactaa gtatcatagc agcagaaagc 10260
   cataagattt taattcatta tctctcattc ctgaacatgc ctccactcac ccacccacat 10320
   acctatgaac agagttaaag tcaaacatac atcaatgtgc atatgatact attccactgc 10380
   atacaggaac tcctacctga atcaagacat atcccctttt tattcctaca gtggggccct 10440
   cgtggaggta aagtgcctga atttcaggaa actacaagag atatatttca tcgagacaaa 10500
   gaaattctta ctgattggaa agagcaatat atgtgtgaag gttggagaaa agagtctaac 10560
   ctgcaaaaaa atagacctaa ccactatagg taagaagttg tatataaaag tatggttgtc 10620
   acttttgggc tacctgaaaa cactgtgtct ggacattctg taggttaaaa gtagacaaat 10680
   agtggaaaca actggcaata gataatagct aattccctac tgtaaatttt tataataaat 10740
   gaaaagcttg aaatttatac tttcctgcag tgaaagaatt ctgaggatct tcaaacccag 10800
   gtgtgaaaga tagtgtttgt gcaaacctac atgaagtggc taactggagc tgggcttcct 10860
   gtcatccatc acaggtgtcc tttccttcct tatctgtcct ttccttcctt acctgtcctt 10920
   ctcccaaatt ccttgtggtc ttctccccaa atccccacaa cattctgagt aagtttagct 10980
   aacttatcaa gttattttaa aaagcatata tgccttctct attagtcaga gttttctaca 11040
   aaaaaaaaag ggaatcaata ggaggataga tagatagatc attgatagga gagatttcta 11100
   ttaagaaatt gacttttgtg gttgtgggaa ctggcaatcg caaaaatcca taaggcaagc 11160
   cagtaggcta gaaattcagg aaagagtgca gtattgagcc taaattccgc agggcaagaa 11220
   actcaagcag attttctgta ttgtactctt gagacagact tgcttcttct tcagggaacc 11280
   tctgtctttg ctctagaggc cttctactga tgaggtgatg cccaccacat cacggaaggc 11340
   aatctacttt actcaaagtt tactgattta aatgttaacc atgtcttaaa aatactttta 11400
   gcattcccta ttcgctcccc cttcaaccct caaaaagaaa attaaaggta agagagcaat 11460
   actcattaga gataagaaag agtaagaaac ctagctcagc tttgtctcag ttttgtttca 11520
   ctaagatgat aaaatagaga ggtaaagcag aagttccatg tgtgaacaat taacttgtga 11580
   aaaggcaaat gtagtagaaa agagacatta ggcagatggc tgtgcatgtt ggccacacag 11640
   aagcagcatt ggccatgacc agtgtgggtc ctggttaggg gaagagaact ggctttgaca 11700
   acaacagggt atctctgagg ttataaaaag ttgggttctg atcatttgga gatgaggtcc 11760
   ctatggatag ggcaccatat ctaaaggttc accatttaca ttgcaaatat acattcagtt 11820
   ctctgagagt gagcagagaa ggcagaggtt ctcagtcttc tgacaaggtc ctggagcatc 11880
   aggggagagc ccattcttac aaaactccac accagcatgc aagcccttac atgcacataa 11940
   gcactcacaa cacaccaaga gcctccaggt gacatctgcc acctccaaat ccccatatcc 12000
   cacatgctca atgcacttgc agtctccatc ccccagcaga ctgcaaatct gacatgcctc 12060
   ctccgaacgg caagggggag aggtacgtat ggtacacaca ctgctgatgg cataggcccc 12120
   tttggaaggg gtagtgtgag tctcttgggg ctatggcaag cacccctgga caagcaggaa 12180
   gagaggtggt ggaggcatgt ctcacggtag catctccttc taggtcctaa tgggacactt 12240
   cattaatgga actaccattt aagtgagttt aaactggatg cttctgattg agccccagag 12300
   ccagtgctcc actgccacca cctgcaccct cacttcccct tgtttaagca tcttccaacc 12360
   cagtaaggct gaagagggaa gcatcctgcc ttcccacttc tcttagcaga gtagattgat 12420
   atgattattc agattgtaca agaatctatt ccctctgaag tattgcttga tgaatgagcc 12480
   cctttttcta atttgctcaa agaaatcatt tgagcttgag gaaaactgtc cagagggcac 12540
   gaggaccagc cgttgtgata tgtaacaagg tagagaaaca aaagctaaat gaagaagagt 12600
   gagcctcaga atcaaagaac tggatttgga tccctttaaa ccattttaca ggggcctgaa 12660
   tgtaattaac ttctctgaaa ttcagtttcc ttatcaatat gctggtgata agtgactatt 12720
   gtttgaagac agcataagca aagcatgcag tacttaggag atgtgttctt ccttcaattc 12780
   ctctattatt aaaagatggg cacagggcag gggcttcagc tcagaaggcc ttgttgagaa 12840
   tggaatggag agcaggaaca agagagaggg gcaaaggcat tgccagcatt ctctgttcgg 12900
   ctgttctcca cccactgcct ttcctcctgc ttccctctaa gtccagggca ttttcccttt 12960
   tgataaactt ccccttttac aacccatcca agggtgaaaa acaaagtcat tacttttttt 13020
   tcagtacctc taaggcaaag cagcagaaac aggcagtcac cactacgaat aagtgactac 13080
   aacaagagct aggccaaact ctgccatgtg ggctgcattt tattgggccg gcaagtaact 13140
   ttaaatccca gctcacactc tactgagtga aagtctgatg aacccgcatc ttcttgtgaa 13200
   caactgcgcc tgagatcagt catgcaagaa gtagcacccc cacccccaga caactaactt 13260
   cccaggctgt gaccaacaag cagccaagag gccaggacag ggaagtctca ggacctttct 13320
   aggaaatcaa tacctttctc tgggtttgtt ctgcctgaaa taataccaat ctccctccaa 13380
   cagcttagca tgtgtggagc atttgatact aacagcaacc ctgcaaggca ggaaggcagt 13440
   agggagaggc ccaagaggaa ttcagcatta aggcagtgag actgacagag gggaccccct 13500
   gaggacattc tggaaggtct tagccagggc caggatgcag acccttcatg tcactgtagc 13560
   tgagacgagg tgcaaggttc acagcatata acctaatttt attacaagaa taaagactca 13620
   gagtttaaat actcctgctt tggggctcat tagtaacaag ttctccaata ttcaaaaggc 13680
   aaagtggatg tgttttagtg taaaattaac actagctgct gtaacaaata agcccccaaa 13740
   catatgatat ctcaaacacc gtaggtttat ttctcactca catcagagtc aaaatggatg 13800
   tttctaacct gcagctgggg cttctcccag cagtattagg ggcactttcc atcttgtggc 13860
   tccaccgtct gtaatgcagg actccaagtg gtggaagagg acggagcaga ggagtcacac 13920
   atgggtgtgt gtctggccca gggtggaagt ggatgtgcat ttcttctgcc cacctcactc 13980
   acaaggccac accccactgc aagagaggct ggagaatgcg gactggattt aaacccaaga 14040
   agaagaaatg gttttctgaa tagttggcca tttactgaca caaaaagggt caaagtgact 14100
   tgcagaggag atgaatttta aatactataa ttatttcctt ggctgccctt tagacagaat 14160
   ttatttcttt ttcttttcca gttaaacctg aggctccttt tgacctgagt gtcgtctatc 14220
   gggaaggagc caatgacttt gtggtgacat ttaatacatc acacttgcaa aagaagtatg 14280
   taaaagtttt aatgcacgat gtagcttacc gccaggaaaa ggatgaaaac aaatggacgg 14340
   tatgtagttc aactacatta ataaaataaa aacttatgaa tgttttctat tttgttggcc 14400
   tagtagtgca tttcccctgg gagggcccaa caattttgct ttcaaaatct accttctact 14460
   gaaagaatct cccaatattg gccccatgaa aacctggatc ttccctgatg catactcttc 14520
   tagctctggt tgttttcttc tgctctaatt ttggtcttca gaatgtttct acattagtga 14580
   gttggataac aatatagatt gaggccaaat taatcctctg tattcagggg cctcaaaaag 14640
   tgtcatgtct agtgccactt tcataggcaa atcaggcaaa atgtatatct gcttatgatc 14700
   accaagtcgt agccacattc tggcttatga gattcatggg accagcatga ggtaaagaaa 14760
   agaggcataa tgtttgcctt tgttttgttt ttattttaaa gcccaaggtc tttgtttttg 14820
   aagtaacagc ttaattttta cccttcataa tcaggagagt tacttagatg ctctcttcat 14880
   gatttgttga ggttggaatg atttggcagt ccctgaaatt tattttgggg aggaggtggc 14940
   agaagagtgg agtgtaccag gttatgagat ttctcttaac ccaccaacct aacttctgtt 15000
   ctttctgcac ctcagagatg aagaagagat gatgatttct cttcctcaag tccttcttat 15060
   tcttgctgtc ctgttttttc aggccaagat tggccttgtt tgtttgcagt gtgatgcaag 15120
   atgccacttg cataaatgta acaactgccc caaaccacct gctccctcct tctactcacc 15180
   caccccaccc ttgatcctgc catctttcat tattcatctg aaaattgcac caattgaaaa 15240
   gcaacttagt ggagaaagga aggattatga ataaatgctg ccaggacaat tagttaacta 15300
   aaaagaaaaa tagataaatt caataaatac atgaattttt ttgagatgga gtcttgctca 15360
   gtcatccagg ttggagtgca atggcgccat cttggctcac tgcaacctcc gcctcccggg 15420
   ttcaagcaat tctcccatct cagcctccca agtacctgtg attacaggca cccgccatca 15480
   tgcccggcta atttttgtat ttttgtagag ctggggtttc accatgttgg ccaggctggt 15540
   cttgaactcc tcacctcagg tgatctgccc acctcagcct cccaaagtgc tgggattaca 15600
   ggcataagcc aacacgccag ccaaaaattg ttttaattaa aaaaaattaa actaaatgcc 15660
   tagccacctt catataacaa caacaaaata ccagatgatt taaggaaatt atataaaagt 15720
   gaaactctaa acaaattaga aaaattatag ccaaatgttt acataatctt gacatgaaga 15780
   agaacattct aagcatcaaa gctgtagaag aaaagaaagg attgagacat gcaactacat 15840
   aaaaagtgga ggtttatata tgtcaacaca cacaataatc aaaaatcaaa aatgcaaatt 15900
   taaaagtaag cttaaattgc cacataaaca gctgatagat ggttagtatc attaatagat 15960
   aaaggactct tataaatcat taaaaaaaca aatatcacaa tagaaaaatg agcaaaaaaa 16020
   ttgggaaaaa tctcataaag tatggaatag ataaattcaa taaatatatg aaaatgaact 16080
   aattatcaaa taaatacaga tataaatagc aatggacttc tttttatctg tcaaattgat 16140
   agagtggttt ttttttaatc ttaaagataa tacactgtgt ggtggagact tttgtctctt 16200
   tatcactatt cacaatgtaa aatggcgtct ttctggagag aaatgattcc tgctcactaa 16260
   cctaacctaa cctttcatct ccccttaata tgtgaaagga tagagagaaa agaagaagat 16320
   attgaagtgt ggaaagggag atcctgggca gtgcctaact cacctgaata agacccatca 16380
   tttcactctc ctccttgacc actcacaaca tcctttataa gctcagattc tgtccctaat 16440
   tttgctgttg actcctttac gtatcagagc tccttattct aacaaatacg agacaacttc 16500
   agagaatgct tatgggacta aaggaatccc aattgaaatg atttgggaga tttaggcaac 16560
   acctcttttc ccatcctaag aatgtaactg cactctactc tctagcatgt gaatttatcc 16620
   agcacaaagc tgacactcct gcagagaaag ctccaaccgg cagcaatgta tgagattaaa 16680
   gttcgatcca tccctgatca ctattttaaa ggcttctgga gtgaatggag tccaagttat 16740
   tacttcagaa ctccagagat caataatagc tcaggtaagg aatggtggta gagtttttgt 16800
   tccctcagag tgctttgcat gtcaaagtgt gggagcaagt gagaggaaga ttgttgaaac 16860
   taacctgcaa aataggacac ccttggaggg cactcttaca ctttctttgg agaatgactt 16920
   gcctgctgtc tttgcgcctt ttgtgaagaa caaggaagca gagggagtgg ggtccttatt 16980
   agctgagaat tagtacaagc catctgtatt cctggaagct gccatacatt ttgaacaaaa 17040
   tccccaccca ctacgtccag ttaaccaatt tagcctggga ccccaatggc tgctgtctct 17100
   aaggcccctt taagaagcac ctttattggt gtcaggtatg caggcaagtg cggctgtcct 17160
   atgtctcctt ttccagaagg atgaagatgt ctttgggact ggaactgaga atgtgtagga 17220
   actgagacat ctcctcccta aaatttgcaa caggggtgaa catccctctc atcatctcct 17280
   gctctggctt cttttccttg gtagaaagtc aagaagggaa gagagcattg gtacctttga 17340
   tgctagatca cgtttacatt tcaagtggca gatgctctgg gcctggtcac ccaagtcaat 17400
   gccttttaaa ccaaaatccc tccataaagc tgtcaaatat gtctcttaac tgaaaagcaa 17460
   ctttcaggaa ataataagtg ggcccacatt actaagtaaa tgcaaagcac cctgagaccc 17520
   tacccccact gcatggctac tgaatgctca ccacaatcta ttcttgcttt ccaggggaga 17580
   tggatcctat cttactaacc atcagcattt tgagtttttt ctctgtcgct ctgttggtca 17640
   tcttggcctg tgtgttatgg aaaaaaaggt gaccttcttc aactaataaa gagggtgatt 17700
   gtgtgggatc acggacagtc agagcttaag ccccatttat tgatgagaaa accacaaagg 17760
   ggattaaggc atttcacgaa tttagtgccc agtatcccta tctatcctca gcgaatttcc 17820
   acagttaatt tcataagagg caaaaagata ttaactggac attaggcaaa cgctgtcccc 17880
   aaagtaagaa ttccgtaatg caatgtttcc caagcttttc ttccagtatc tcctaaaggt 17940
   tgcagaagcc atcattcatg gcaaatgcac aatgtacttg aagtcttgac tttaagcata 18000
   taaattcatg ggatttttta tactaatcca tgctaaggct atgtttgttt catcataaaa 18060
   atgatgtttt aaacatgcac ttgttaaatt acttactatt ctaggagaat gcccttggca 18120
   gaaggctaca tatccccagg attacaaata ccctaatttg aaaagtactg cctggaaagt 18180
   actaagcaat ttccctgggt aatttgaaaa tattccccca cttccaccaa aattagctgc 18240
   cagagttgct gtcagtaaag agaagaaata aaaagacaac acttaaattg ggagtaaaca 18300
   acggggttaa atttacttcc atagctgcac caaaattacc tccttgcaag ccttggtgtt 18360
   ccttccctct agggcttttt cccagaggta tattattgtc atgtcttgtt cacagaatgg 18420
   attgatatct gtggtctctg gtccaacccc tccttgaatt gatagggccc cgaggcccag 18480
   agaaagccag tctcttgacc atggtcaccc acctaattgt gttagagcca agactagaaa 18540
   tctgttcttc tgattccaag ctcagaataa gtgggaagac tcagtgtgcc tgtgccctct 18600
   gccattcact tcatctatca atgttctctg atttcaggat taagcctatc gtatggccca 18660
   gtctccccga tcataagaag actctggaac atctttgtaa gaaaccaaga aaagtgagtg 18720
   tttttggtgc ttaaaaagtg ttgtgttggc aacatcccag tggccaagaa tgatattcca 18780
   ggacaaggaa cagttgaacc tcaccttttg gtatttgatt catcctgtaa ctagggtccc 18840
   tcctaagacc ctagctgcag tagggaactg aaataagata cacatctcag aacttctggg 18900
   ctccctgggg ctggagggca cagccagtgg tcacttcaag tcttgaagtg tctcagaagc 18960
   tccagaagca aagagtccat tgaggaacat gctggcaatt ctgtgacatt ccctgtcaga 19020
   aaactctata gacctactcc tgaactgaac atttgatggt gtgtctctct ggtgccatct 19080
   taataccctt tctccttttt ctgtgcagaa tttaaatgtg agtttcaatc ctgaaagttt 19140
   cctggactgc cagattcata gggtggatga cattcaagct agagatgaag tggaaggttt 19200
   tctgcaagat acgtttcctc agcaactaga agaatctgag aagcagaggc ttggagggga 19260
   tgtgcagagc cccaactgcc catctgagga tgtagtcatc actccagaaa gctttggaag 19320
   agattcatcc ctcacatgcc tggctgggaa tgtcagtgca tgtgacgccc ctattctctc 19380
   ctcttccagg tccctagact gcagggagag tggcaagaat gggcctcatg tgtaccagga 19440
   cctcctgctt agccttggga ctacaaacag cacgctgccc cctccatttt ctctccaatc 19500
   tggaatcctg acattgaacc cagttgctca gggtcagccc attcttactt ccctgggatc 19560
   aaatcaagaa gaagcatatg tcaccatgtc cagcttctac caaaaccagt gaagtgtaag 19620
   aaacccagac tgaacttacc gtgagcgaca aagatgattt aaaagggaag tctagagttc 19680
   ctagtctccc tcacagcaca gagaagacaa aattagcaaa accccactac acagtctgca 19740
   agattctgaa acattgcttt gaccactctt cctgagttca gtggcactca acatgagtca 19800
   agagcatcct gcttctacca tgtggatttg gtcacaaggt ttaaggtgac ccaatgattc 19860
   agctatttaa aaaaaaaaga ggaaagaatg aaagagtaaa ggaaatgatt gaggagtgag 19920
   gaaggcagga agagagcatg agaggaaaga aagaaaggaa aataaaaaat gatagttgcc 19980
   attattagga tttaatatat atccagtgct ttgcaagtgc tctgcgcacc ttgtctcact 20040
   ccatcctgac aataatcctg ggaggtgtgt gcaattacta cgactactct cttttttata 20100
   gatcattaaa ttcagaacta aggagttaag taacttgtcc aagttgttca cacagtgaag 20160
   ggaggggcca agatatgatg gctgggagtc taattgcagt tccctgagcc atgtgccttt 20220
   ctcttcactg aggactgccc cattcttgag tgccaaacgt cactagtaac agggtgtgcc 20280
   tagataattt atgatccaaa ctgagtcagt ttggaaagtg aaagggaaac ttacatataa 20340
   tccctccggg acaatgagca aaaactagga ctgtccccag acaaatgtga acatacatat 20400
   catcacttaa attaaaatgg ctatgagaaa gaaagagggg gagaaacagt cttgcgggtg 20460
   tgaagtccca tgaccagcca tgtcaaaaga aggtaaagaa gtcaagaaaa agccatgaag 20520
   cccatttggt ttcatttttc tgaaaatagg ctcaagaggg aataaattag aaactcacaa 20580
   tttctcttgt ttgttaccaa gacagtgatt ctcttgctgc taccacccaa ctgcatccgt 20640
   ccatgatctc agaggaaact gtcgctgacc ctggacatgg gtacgtttga cgagtgagag 20700
   gaggcatgac ccctcccatg tgtatagaca ctaccccaac ctaaattcat ccctaaattg 20760
   tcccaagttc tccagcaata gaggctgcca caaacttcag ggagaaagag ttacaagtac 20820
   atgcaatgag tgaactgact gtggctacaa tcttgaagat atacggaaga gacgtattat 20880
   taatgcttga catatatcat cttgcctttc ttggtctaga ctgacttcta atgactaact 20940
   caaagtcaag gcaactgagt aatgtcagct cagcaaagtg cagcaaaccc atctcccaca 21000
   ggcctccaaa ccctggctgt tcacagaacc acaaagggca gatgctgcac agaaaactag 21060
   agaaggggtc ataggttcat ggttttgttt gagatttgtt gctactgttt ttctgttttg 21120
   aattttcttc tttgttctgt ttttacttta tttaggggga ctaggtgttt ctgatatttt 21180
   agttttcttg tttgttttgt tttgtgttgt ctgtgaatgg ggttttaact gtggatgaat 21240
   ggaccttatc tgttggctta aaggactggt aagatcagac catcttattc ttcaggtgaa 21300
   tgttttactt tccaaagtgc tctcctctgc accagcagta ataaatacaa tgccataatc 21360
   ccttaggttt gcctagtgct tttgcaattt tcaaagcact tccataagca ttccttccac 21420
   ctccttgata ggcatttatg gaaagcctgc tacatgtcaa tcatactgtt aggcacaggg 21480
   gacctaaaga cacataaaag gatggcattc tgcctcataa attgcaaaac ctaatgaaag 21540
   tgactgcttg gtaaacaaat tattattata ttataaaatg ctataaaaga gccatattga 21600
   aagtgccctg ttggagacag ggcaaatgcc acaaaaatga tgtaaattta catggaggaa 21660
   aagtagaatc tgcctggttt gtaggcagca gaagacattt ttcatcagtg ggcaggtgtt 21720
   ctttaccttt tgtagaaatg ggagtcaagt ctcaaatagg aggctccaca aaatctcatg 21780
   ccaggtctct gataccttat tcacagaagt tctttgaagt atttattgtt attttctttg 21840
   acttatggga aaactgggac acaggaagac aggtaaatta cccaacctca cacgttaagt 21900
   cagaactggg agccataatt ttgtatccct ggtataaata gacaatctct tgaagaaatg 21960
   aagagatgac catagaaaaa catcgagata tctccagctc taaaatcctt tgtttcaatg 22020
   ttgtttggca tatgttatct ttggaattta gtgtctgagc ctctgtctgt tactgtagta 22080
   tttaaaatgc atgtattata atcatataat cataactgct gttaattctt gattatatac 22140
   ctagggacaa tgtgtaatgt aagattacta attggttctg cccaatctcc tttcagattt 22200
   tattaggaaa aaaaaataaa cctcctgatc ggagacaatg tattaatcag aagtgtaaac 22260
   tgccagttct atatagcatg aaatgaaaag acagctaatt tggtccaaca aacatgactg 22320
   ggtctagggc acccaggctg attcagctga tttcctacca gcctttgcct cttccttcaa 22380
   tgtggtttcc atgggaattt gcttcagaaa agccaagtat gggctgttca gaggtgcaca 22440
   cctgcatttt cttagctctt ctagaggggc taagagactt ggtacgggcc aggaagaata 22500
   tgtggcagag ctcctggaaa tgatgcagat taggtggcat ttttgtcagc tctgtggttt 22560
   attgttggga ctattcttta aaatatccat tgttcactac agtgaagatc tctgatttaa 22620
   ccgtgtacta tccacatgca ttacaaacat ttcgcagagc tgcttagtat ataagcgtac 22680
   aatgtatgta ataaccatct catatttaat taaatggtat agaagaaca 22729
<210> 2
   <211> 4626
   <212> RNA
   <213> Artificial Sequence
<220>
   <223> IL7R-001 transcript
<400> 2
   gtggttagat aagtataaag ccctagatct aagcttctct gtcttcctcc ctccctccct      60
   tcctcttact ctcattcatt tcatacacac tggctcacac atctactctc tctctctatc     120
   tctctcagaa tgacaattct aggtacaact tttggcatgg ttttttcttt acttcaagtc     180
   gtttctggag aaagtggcta tgctcaaaat ggagacttgg aagatgcaga actggatgac     240
   tactcattct catgctatag ccagttggaa gtgaatggat cgcagcactc actgacctgt     300
   gcttttgagg acccagatgt caacatcacc aatctggaat ttgaaatatg tggggccctc     360
   gtggaggtaa agtgcctgaa tttcaggaaa ctacaagaga tatatttcat cgagacaaag     420
   aaattcttac tgattggaaa gagcaatata tgtgtgaagg ttggagaaaa gagtctaacc     480
   tgcaaaaaaa tagacctaac cactatagtt aaacctgagg ctccttttga cctgagtgtc     540
   gtctatcggg aaggagccaa tgactttgtg gtgacattta atacatcaca cttgcaaaag     600
   aagtatgtaa aagttttaat gcacgatgta gcttaccgcc aggaaaagga tgaaaacaaa     660
   tggacgcatg tgaatttatc cagcacaaag ctgacactcc tgcagagaaa gctccaaccg     720
   gcagcaatgt atgagattaa agttcgatcc atccctgatc actattttaa aggcttctgg     780
   agtgaatgga gtccaagtta ttacttcaga actccagaga tcaataatag ctcaggggag     840
   atggatccta tcttactaac catcagcatt ttgagttttt tctctgtcgc tctgttggtc 900
   atcttggcct gtgtgttatg gaaaaaaagg attaagccta tcgtatggcc cagtctcccc 960
   gatcataaga agactctgga acatctttgt aagaaaccaa gaaaaaattt aaatgtgagt 1020
   ttcaatcctg aaagtttcct ggactgccag attcataggg tggatgacat tcaagctaga 1080
   gatgaagtgg aaggttttct gcaagatacg tttcctcagc aactagaaga atctgagaag 1140
   cagaggcttg gaggggatgt gcagagcccc aactgcccat ctgaggatgt agtcatcact 1200
   ccagaaagct ttggaagaga ttcatccctc acatgcctgg ctgggaatgt cagtgcatgt 1260
   gacgccccta ttctctcctc ttccaggtcc ctagactgca gggagagtgg caagaatggg 1320
   cctcatgtgt accaggacct cctgcttagc cttgggacta caaacagcac gctgccccct 1380
   ccattttctc tccaatctgg aatcctgaca ttgaacccag ttgctcaggg tcagcccatt 1440
   cttacttccc tgggatcaaa tcaagaagaa gcatatgtca ccatgtccag cttctaccaa 1500
   aaccagtgaa gtgtaagaaa cccagactga acttaccgtg agcgacaaag atgatttaaa 1560
   agggaagtct agagttccta gtctccctca cagcacagag aagacaaaat tagcaaaacc 1620
   ccactacaca gtctgcaaga ttctgaaaca ttgctttgac cactcttcct gagttcagtg 1680
   gcactcaaca tgagtcaaga gcatcctgct tctaccatgt ggatttggtc acaaggttta 1740
   aggtgaccca atgattcagc tatttaaaaa aaaaagagga aagaatgaaa gagtaaagga 1800
   aatgattgag gagtgaggaa ggcaggaaga gagcatgaga ggaaagaaag aaaggaaaat 1860
   aaaaaatgat agttgccatt attaggattt aatatatatc cagtgctttg caagtgctct 1920
   gcgcaccttg tctcactcca tcctgacaat aatcctggga ggtgtgtgca attactacga 1980
   ctactctctt ttttatagat cattaaattc agaactaagg agttaagtaa cttgtccaag 2040
   ttgttcacac agtgaaggga ggggccaaga tatgatggct gggagtctaa ttgcagttcc 2100
   ctgagccatg tgcctttctc ttcactgagg actgccccat tcttgagtgc caaacgtcac 2160
   tagtaacagg gtgtgcctag ataatttatg atccaaactg agtcagtttg gaaagtgaaa 2220
   gggaaactta catataatcc ctccgggaca atgagcaaaa actaggactg tccccagaca 2280
   aatgtgaaca tacatatcat cacttaaatt aaaatggcta tgagaaagaa agagggggag 2340
   aaacagtctt gcgggtgtga agtcccatga ccagccatgt caaaagaagg taaagaagtc 2400
   aagaaaaagc catgaagccc atttggtttc atttttctga aaataggctc aagagggaat 2460
   aaattagaaa ctcacaattt ctcttgtttg ttaccaagac agtgattctc ttgctgctac 2520
   cacccaactg catccgtcca tgatctcaga ggaaactgtc gctgaccctg gacatgggta 2580
   cgtttgacga gtgagaggag gcatgacccc tcccatgtgt atagacacta ccccaaccta 2640
   aattcatccc taaattgtcc caagttctcc agcaatagag gctgccacaa acttcaggga 2700
   gaaagagtta caagtacatg caatgagtga actgactgtg gctacaatct tgaagatata 2760
   cggaagagac gtattattaa tgcttgacat atatcatctt gcctttcttg gtctagactg 2820
   acttctaatg actaactcaa agtcaaggca actgagtaat gtcagctcag caaagtgcag 2880
   caaacccatc tcccacaggc ctccaaaccc tggctgttca cagaaccaca aagggcagat 2940
   gctgcacaga aaactagaga aggggtcata ggttcatggt tttgtttgag atttgttgct 3000
   actgtttttc tgttttgaat tttcttcttt gttctgtttt tactttattt agggggacta 3060
   ggtgtttctg atattttagt tttcttgttt gttttgtttt gtgttgtctg tgaatggggt 3120
   tttaactgtg gatgaatgga ccttatctgt tggcttaaag gactggtaag atcagaccat 3180
   cttattcttc aggtgaatgt tttactttcc aaagtgctct cctctgcacc agcagtaata 3240
   aatacaatgc cataatccct taggtttgcc tagtgctttt gcaattttca aagcacttcc 3300
   ataagcattc cttccacctc cttgataggc atttatggaa agcctgctac atgtcaatca 3360
   tactgttagg cacaggggac ctaaagacac ataaaaggat ggcattctgc ctcataaatt 3420
   gcaaaaccta atgaaagtga ctgcttggta aacaaattat tattatatta taaaatgcta 3480
   taaaagagcc atattgaaag tgccctgttg gagacagggc aaatgccaca aaaatgatgt 3540
   aaatttacat ggaggaaaag tagaatctgc ctggtttgta ggcagcagaa gacatttttc 3600
   atcagtgggc aggtgttctt taccttttgt agaaatggga gtcaagtctc aaataggagg 3660
   ctccacaaaa tctcatgcca ggtctctgat accttattca cagaagttct ttgaagtatt 3720
   tattgttatt ttctttgact tatgggaaaa ctgggacaca ggaagacagg taaattaccc 3780
   aacctcacac gttaagtcag aactgggagc cataattttg tatccctggt ataaatagac 3840
   aatctcttga agaaatgaag agatgaccat agaaaaacat cgagatatct ccagctctaa 3900
   aatcctttgt ttcaatgttg tttggcatat gttatctttg gaatttagtg tctgagcctc 3960
   tgtctgttac tgtagtattt aaaatgcatg tattataatc atataatcat aactgctgtt 4020
   aattcttgat tatataccta gggacaatgt gtaatgtaag attactaatt ggttctgccc 4080
   aatctccttt cagattttat taggaaaaaa aaataaacct cctgatcgga gacaatgtat 4140
   taatcagaag tgtaaactgc cagttctata tagcatgaaa tgaaaagaca gctaatttgg 4200
   tccaacaaac atgactgggt ctagggcacc caggctgatt cagctgattt cctaccagcc 4260
   tttgcctctt ccttcaatgt ggtttccatg ggaatttgct tcagaaaagc caagtatggg 4320
   ctgttcagag gtgcacacct gcattttctt agctcttcta gaggggctaa gagacttggt 4380
   acgggccagg aagaatatgt ggcagagctc ctggaaatga tgcagattag gtggcatttt 4440
   tgtcagctct gtggtttatt gttgggacta ttctttaaaa tatccattgt tcactacagt 4500
   gaagatctct gatttaaccg tgtactatcc acatgcatta caaacatttc gcagagctgc 4560
   ttagtatata agcgtacaat gtatgtaata accatctcat atttaattaa atggtataga 4620
   agaaca 4626
<210> 3
   <211> 1287
   <212> RNA
   <213> Artificial Sequence
<220>
   <223> IL7R-002 transcript
<400> 3
   gtcttcctcc ctccctccct tcctcttact ctcattcatt tcatacacac tggctcacac      60
   atctactctc tctctctatc tctctcagaa tgacaattct aggtacaact tttggcatgg     120
   ttttttcttt acttcaagtc gtttctggag aaagtggcta tgctcaaaat ggagacttgg     180
   aagatgcaga actggatgac tactcattct catgctatag ccagttggaa gtgaatggat     240
   cgcagcactc actgacctgt gcttttgagg acccagatgt caacatcacc aatctggaat     300
   ttgaaatatg tggggccctc gtggaggtaa agtgcctgaa tttcaggaaa ctacaagaga     360
   tatatttcat cgagacaaag aaattcttac tgattggaaa gagcaatata tgtgtgaagg     420
   ttggagaaaa gagtctaacc tgcaaaaaaa tagacctaac cactatagtt aaacctgagg     480
   ctccttttga cctgagtgtc gtctatcggg aaggagccaa tgactttgtg gtgacattta     540
   atacatcaca cttgcaaaag aagtatgtaa aagttttaat gcacgatgta gcttaccgcc     600
   aggaaaagga tgaaaacaaa tggacggatt aagcctatcg tatggcccag tctccccgat     660
   cataagaaga ctctggaaca tctttgtaag aaaccaagaa aaaatttaaa tgtgagtttc     720
   aatcctgaaa gtttcctgga ctgccagatt catagggtgg atgacattca agctagagat     780
   gaagtggaag gttttctgca agatacgttt cctcagcaac tagaagaatc tgagaagcag     840
   aggcttggag gggatgtgca gagccccaac tgcccatctg aggatgtagt catcactcca     900
   gaaagctttg gaagagattc atccctcaca tgcctggctg ggaatgtcag tgcatgtgac     960
   gcccctattc tctcctcttc caggtcccta gactgcaggg agagtggcaa gaatgggcct    1020
   catgtgtacc aggacctcct gcttagcctt gggactacaa acagcacgct gccccctcca    1080
   ttttctctcc aatctggaat cctgacattg aacccagttg ctcagggtca gcccattctt    1140
   acttccctgg gatcaaatca agaagaagca tatgtcacca tgtccagctt ctaccaaaac    1200
   cagtgaagtg taagaaaccc agactgaact taccgtgagc gacaaagatg atttaaaagg    1260
   gaagtctaga gttcctagtc tccctca                                        1287
<210> 4
   <211> 1004
   <212> RNA
   <213> Artificial Sequence
<220>
   <223> IL7R-003 transcript
<400> 4
   cttcctccct ccctcccttc ctcttactct cattcatttc atacacactg gctcacacat      60
   ctactctctc tctctatctc tctcagaatg acaattctag gtacaacttt tggcatggtt     120
   ttttctttac ttcaagtcgt ttctggagaa agtggctatg ctcaaaatgg agacttggaa     180
   gatgcagaac tggatgacta ctcattctca tgctatagcc agttggaagt gaatggatcg     240
   cagcactcac tgacctgtgc ttttgaggac ccagatgtca acatcaccaa tctggaattt     300
   gaaatatgtg gggccctcgt ggaggtaaag tgcctgaatt tcaggaaact acaagagata     360
   tatttcatcg agacaaagaa attcttactg attggaaaga gcaatatatg tgtgaaggtt     420
   ggagaaaaga gtctaacctg caaaaaaata gacctaacca ctatagttaa acctgaggct     480
   ccttttgacc tgagtgtcgt ctatcgggaa ggagccaatg actttgtggt gacatttaat     540
   acatcacact tgcaaaagaa gtatgtaaaa gttttaatgc acgatgtagc ttaccgccag     600
   gaaaaggatg aaaacaaatg gacgcatgtg aatttatcca gcacaaagct gacactcctg     660
   cagagaaagc tccaaccggc agcaatgtat gagattaaag ttcgatccat ccctgatcac     720
   tattttaaag gcttctggag tgaatggagt ccaagttatt acttcagaac tccagagatc     780
   aataatagct caggattaag cctatcgtat ggcccagtct ccccgatcat aagaagactc     840
   tggaacatct ttgtaagaaa ccaagaaaag tgagtgtttt tggtgcttaa aaagtgttgt     900
   gttggcaaca tcccagtggc caagaatgat attccaggac aaggaacagt tgaacctcac     960
   cttttggtat ttgattcatc ctgtaactag ggtccctcct aaga                     1004
<210> 5
   <211> 562
   <212> RNA
   <213> Artificial Sequence
<220>
   <223> IL7R-004 transcript
<400> 5
   aaatgctaga gactattcat ttaggaccag agtggcatta gctttcctac agccatcctc 60
   acctgcttga cacccttgga tgttgggttt ttttgtttgt ttgtttgttt gtttgttgtt 120
   ttttttttcc acagctcttg tggtccccag cctctctctt gagcagggct ggcttttttt 180
   ttttaataag atagctggtg cccaagattg ttttccacct taaggataaa acctgttaag 240
   aaaggtcata gtttgccagc ccctgcccta aacaaataat tcttgaatgc ctactgtggt 300
   gtgtaagata tgagtaaata ccagggatac acagagaaca aaagagaaaa actgctattc 360
   ttgtgaaact tggaagttgg agaatgacaa ttctaggtac aacttttggc atggtttttt 420
   ctttacttca agtcgtttct ggagaaagtg gctatgctca aaatggagac ttggaagatg 480
   cagaactgga tgactactca ttctcatgct atagccagtt ggaagtgaat ggatcgcagc 540
   actcactgac ctgtgctttt ga 562
<210> 6
   <211> 515
   <212> RNA
   <213> Artificial Sequence
<220>
   <223> IL7R-005 transcript
<400> 6
   agagtggcat tagctttcct acagccatcc tcacctgctt gacacccttg gatgttgggt 60
   tttttgtttg tttgtttgtt tgtttgttgt tttttttttc cacagctctt gtggtcccca 120
   gcctctctct tgagcagggc tggctttttt ttttttaata agatagctgg tgcccaagat 180
   tgttttccac cttaaggata aaacctgtta agaaaggaga cttggaagat gcagaactgg 240
   atgactactc attctcatgc tatagccagt tggaagtgaa tggatcgcag cactcactga 300
   cctgtgcttt tgaggaccca gatgtcaaca tcaccaatct ggaatttgaa atatgtgggg 360
   ccctcgtgga ggtaaagtgc ctgaatttca ggaaactaca agagatatat ttcatcgaga 420
   caaagaaatt cttactgatt ggaaagagca atatatgtgt gaaggttgga gaaaagagtc 480
   taacctgcaa aaaaatagac ctaaccacta taggt 515
<210> 7
   <211> 551
   <212> RNA
   <213> Artificial Sequence
<220>
   <223> IL7R-006 transcript
<400> 7
   cttttaaagt gggcccttag tcaggagcgg tggctcatgc ctgtagtcct agcactttgg      60
   gaggctgagg caggcagatc acttgaggtc aggagttcga gacaagcctg gccaacatgg     120
   cgaaaccccg tctccactga aaacacaaaa attaggctgg catagtggca tttgcctgta     180
   gtcctagcta ctcaggaggc tgaggcagga gaattgcttg aacctgggag gtggaaattg     240
   cagtgagccg agatcatgct attgtactcc agcctgggca acaaagcaag actctgtctc     300
   aaaaaaataa aaattaaaaa aataaagtag cctctagcct aagatagctt gagcctaggt     360
   gtgaatctac tgccttactc tgatgtaagc acaaatgaca attctaggta caacttttgg     420
   catggttttt tctttacttc aagtcgtttc tggagaaagt ggctatgctc aaaatggaga     480
   cttggaagat gcagaactgg atgactactc attctcatgc tatagccagt tggaagtgaa     540
   tggatcgcag c                                                          551
<210> 8
   <211> 612
   <212> RNA
   <213> Artificial Sequence
<220>
   <223> IL7R-007 transcript
<400> 8
   ttcctccctc cctcccttcc tcttactctc attcatttca tacacactgg ctcacacatc      60
   tactctctct ctctatctct ctcagaatga caattctagg tacaactttt ggcatggttt     120
   tttctttact tcaagtcgtt tctggagaaa gtggctatgc tcaaaatgga gacttggaag     180
   atgcagaact ggatgactac tcattctcat gctatagcca gttggaagtg aatggatcgc     240
   agcactcact gacctgtgct tttgaggacc cagatgtcaa catcaccaat ctggaatttg     300
   aaatatgtgg ggccctcgtg gaggtaaagt gcctgaattt caggaaacta caagagatat     360
   atttcatcga gacaaagaaa ttcttactga ttggaaagag caatatatgt gtgaaggttg     420
   gagaaaagag tctaacctgc aaaaaaatag acctaaccac tataggtaag aagttgtata     480
   taaaagtatg gttgtcactt ttgggctacc tgaaaacact gtgtctggac attctgtagg     540
   ttaaaagtag acaaatagtg gaaacaactg gcaatagata atagctaatt ccctactgta     600
   aatttttata at                                                         612
<210> 9
   <211> 552
   <212> RNA
   <213> Artificial Sequence
<220>
   <223> IL7R-008 transcript
<400> 9
   gtgtggaaag ggagatcctg ggcagtgcct aactcacctg aataagaccc atcatttcac 60
   tctcctcctt gaccactcac aacatccttt ataagctcag attctgtccc taattttgct 120
   gttgactcct ttacgtatca gagctcctta ttctaacaaa tacgagacaa cttcagagaa 180
   tgcttatggg actaaaggaa tcccaattga aatgatttgg gagatttagg caacacctct 240
   tttcccatcc taagaatgta actgcactct actctctagc atgtgaattt atccagcaca 300
   aagctgacac tcctgcagag aaagctccaa ccggcagcaa tgtatgagat taaagttcga 360
   tccatccctg atcactattt taaaggcttc tggagtgaat ggagtccaag ttattacttc 420
   agaactccag agatcaataa tagctcaggg gagatggatc ctatcttact aaccatcagc 480
   attttgagtt ttttctctgt cgctctgttg gtcatcttgg cctgtgtgtt atggaaaaaa 540
   aggattaagc ct 552
<210> 10
   <211> 626
   <212> RNA
   <213> Artificial Sequence
<220>
   <223> IL7R-009 transcript
<400> 10
   atctgttctt ctgattccaa gctcagaata agtgggaaga ctcagtgtgc ctgtgccctc 60
   tgccattcac ttcatctatc aatgttctct gatttcagga ttaagcctat cgtatggccc 120
   agtctccccg atcataagaa gactctggaa catctttgta agaaaccaag aaaaaattta 180
   aatgtgagtt tcaatcctga aagtttcctg gactgccaga ttcatagggt ggatgacatt 240
   caagctagag atgaagtgga aggttttctg caagatacgt ttcctcagca actagaagaa 300
   tctgagaagc agaggcttgg aggggatgtg cagagcccca actgcccatc tgaggatgta 360
   gtcatcactc cagaaagctt tggaagagat tcatccctca catgcctggc tgggaatgtc 420
   agtgcatgtg acgcccctat tctctcctct tccaggtccc tagactgcag ggagagtggc 480
   aagaatgggc ctcatgtgta ccaggacctc ctgcttagcc ttgggactac aaacagcacg 540
   ctgccccctc cattttctct ccaatctgga atcctgacat tgaacccagt tgctcagggt 600
   cagcccattc ttacttccct gggatc 626
<210> 11
   <211> 917
   <212> RNA
   <213> Artificial Sequence
<220>
   <223> IL7R-010 transcript
<400> 11
   gcagcaatgt atgagattaa agttcgatcc atccctgatc actattttaa aggcttctgg      60
   agtgaatgga gtccaagtta ttacttcaga actccagaga tcaataatag ctcaggatta     120
   agcctatcgt atggcccagt ctccccgatc ataagaagac tctggaacat ctttgtaaga     180
   aaccaagaaa aaatttaaat gtgagtttca atcctgaaag tttcctggac tgccagattc     240
   atagggtgga tgacattcaa gctagagatg aagtggaagg ttttctgcaa gatacgtttc     300
   ctcagcaact agaagaatct gagaagcaga ggcttggagg ggatgtgcag agccccaact     360
   gcccatctga ggatgtagtc atcactccag aaagctttgg aagagattca tccctcacat     420
   gcctggctgg gaatgtcagt gcatgtgacg cccctattct ctcctcttcc aggtccctag     480
   actgcaggga gagtggcaag aatgggcctc atgtgtacca ggacctcctg cttagccttg     540
   ggactacaaa cagcacgctg ccccctccat tttctctcca atctggaatc ctgacattga     600
   acccagttgc tcagggtcag cccattctta cttccctggg atcaaatcaa gaagaagcat     660
   atgtcaccat gtccagcttc taccaaaacc agtgaagtgt aagaaaccca gactgaactt     720
   accgtgagcg acaaagatga tttaaaaggg aagtctagag ttcctagtct ccctcacagc     780
   acagagaaga caaaattagc aaaaccccac tacacagtct gcaagattct gaaacattgc     840
   tttgaccact cttcctgagt tcagtggcac tcaacatgag tcaagagcat cctgcttcta     900
   ccatgtggat ttggtca                                                    917
<210> 12
   <211> 20
   <212> DNA
   <213> Artificial Sequence
<220>
   <223> Forward primer for PCR-A
<400> 12
   ggaagtgaat ggatcgcagc 20
<210> 13
   <211> 19
   <212> DNA
   <213> Artificial Sequence
<220>
   <223> Reverse primer for PCR-A
<400> 13
   ggcactttac ctccacgag 19
<210> 14
   <211> 22
   <212> DNA
   <213> Artificial Sequence
<220>
   <223> Probe for PCR-A
<400> 14
   ctgtgctttt gaggacccag at 22
<210> 15
   <211> 19
   <212> DNA
   <213> Artificial Sequence
<220>
   <223> Forward primer for PCR-B
<400> 15
   ctctgtcgct ctgttggtc 19
<210> 16
   <211> 21
   <212> DNA
   <213> Artificial Sequence
<220>
   <223> Reverse primer for PCR-B
<400> 16
   tccagagtct tcttatgatc g 21
<210> 17
   <211> 21
   <212> DNA
   <213> Artificial Sequence
<220>
   <223> Probe for PCR-B
<400> 17
   ctatcgtatg gcccagtctc c 21
<210> 18
   <211> 20
   <212> DNA
   <213> Artificial Sequence
<220>
   <223> Forward primer for PCR-C
<400> 18
   ggaagtgaat ggatcgcagc 20
<210> 19
   <211> 20
   <212> DNA
   <213> Artificial Sequence
<220>
   <223> Reverse primer for PCR-C
<400> 19
   cagaatgtcc agacacagtg 20
<210> 20
   <211> 22
   <212> DNA
   <213> Artificial Sequence
<220>
   <223> Probe for PCR-C
<400> 20
   ctgtgctttt gaggacccag at 22